(19) Europäisches Patentamt

European Patent Office

Office européen des brevets

(11) **EP 2 785 294 B1**

(12) **EUROPEAN PATENT SPECIFICATION**

(45) Date of publication and mention
of the grant of the patent:
**23.11.2016 Bulletin 2016/47**

(21) Application number: **12816806.9**

(22) Date of filing: **30.11.2012**

(51) Int Cl.:
*A61F 9/008* (2006.01)    *A61B 3/107* (2006.01)
*A61B 3/00* (2006.01)    *A61B 5/107* (2006.01)
*A61B 5/00* (2006.01)    *G01B 11/24* (2006.01)

(86) International application number:
**PCT/US2012/067357**

(87) International publication number:
**WO 2013/082466 (06.06.2013 Gazette 2013/23)**

(54) **SYSTEM AND METHOD FOR OPHTHALMIC SURFACE MEASUREMENTS BASED ON SEQUENTIAL ESTIMATES**

SYSTEM UND VERFAHREN FÜR OPHTHALMISCHE OBERFLÄCHENMESSUNGEN AUF DER BASIS VON SEQUENZIELLEN SCHÄTZUNGEN

SYSTÈME ET PROCÉDÉ POUR MESURES DE SURFACE OPHTALMIQUE SUR LA BASE D'ESTIMATIONS SÉQUENTIELLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2011 US 201161565436 P**

(43) Date of publication of application:
**08.10.2014 Bulletin 2014/41**

(73) Proprietor: **AMO Development, LLC**
**Santa Ana, CA 92705 (US)**

(72) Inventor: **FABRIKANT, Anatoly**
**Frement**
**CA 94539 (US)**

(74) Representative: **Hoffmann Eitle**
**Patent- und Rechtsanwälte PartmbB**
**Arabellastraße 30**
**81925 München (DE)**

(56) References cited:
**WO-A1-03/050472        WO-A1-2005/092172**
**WO-A1-2009/136372**

• **THIBOS L N ET AL: "A statistical model of the aberration structure of normal, well-corrected eyes", OPHTHALMIC AND PHYSIOLOGICAL, vol. 22, no. 5, September 2002 (2002-09), pages 427-433, XP002695349, GB ISSN: 0275-5408**
• **WEAAM F. M. ALKHALDI: 'Statistical Signal and Image Processing Techniques in Corneal Modeling', [Online] 13 July 2010, XP055197659 Retrieved from the Internet: <URL:http://tuprints.ulb.tu-darmstadt.de/2232/2/WeaamAlkhaldi_PhD_Thesis.pdf> [retrieved on 2015-06-23]**

**Description**

BACKGROUND OF THE INVENTION

Field of the Invention

The present invention is generally related to surface measurements of an eye and more particularly, corneal topography or wavefront measurements of the eye based on sequential estimation.

Background

Known laser eye surgery procedures generally employ an ultraviolet or infrared laser to remove a microscopic layer of stromal tissue from the cornea of the eye. Examples of laser eye surgery procedures include photorefractive keratectomy (PRK), phototherapeutic keratectomy (PTK), laser assisted in situ keratomileusis (LASIK), laser epithelial keratomileusis (LASEK), and the like. A laser typically removes a selected shape of a corneal tissue, often to correct refractive errors of an eye. Ultraviolet laser ablation results in photodecomposition of a corneal tissue, but generally does not cause significant thermal damage to adjacent and underlying tissues of an eye. Irradiated molecules are broken into smaller volatile fragments photochemically, directly breaking intermolecular bonds.

Laser ablation procedures can remove a targeted amount of corneal stroma to change the corneal contour for varying purposes, such as for correcting myopia, hyperopia, astigmatism, and the like. Control over a distribution of ablation energy across a cornea may be provided by a variety of systems and methods, including use of ablatable masks, fixed and moveable apertures, controlled scanning systems, eye movement tracking mechanisms, and the like. In known systems, a laser beam often comprises a series of discrete pulses of laser light energy, with a total shape and amount of tissue removed being determined by a shape, size, location, and/or number of laser energy pulses impinging on the cornea. A variety of algorithms may be used to calculate the pattern of laser pulses used to reshape the cornea so as to correct a refractive error of an eye. Known systems make use of a variety of forms of lasers and laser energy to effect a correction, including infrared lasers, ultraviolet lasers, femtosecond lasers, wavelength multiplied solid-state lasers, and the like. Alternative vision correction techniques make use of radial incisions in a cornea, intraocular lenses, removable corneal support structures, and the like.

Known corneal correction treatment methods have generally been successful in correcting standard vision errors, such as myopia, hyperopia, astigmatism, and the like. By customizing an ablation pattern based on wavefront measurements, it has been possible to correct minor aberrations so as to reliably and repeatedly provide visual acuity greater than 20/20. Such detailed corrections benefit from an extremely accurate ablation of tissue.

Known methods for calculation of a customized ablation pattern using wavefront sensor data generally involves mathematically modeling a surface of the cornea using expansion series techniques. More specifically, Zernike polynomials have been employed to model the corneal surface and refractive aberrations of the eye. Coefficients of a Zernike polynomial are derived through known fitting techniques, and an optical correction procedure is then determined using a shape indicated by a mathematical series expansion model.

More recently, refractive correction treatments have considered combining corneal topography measurement data with wavefront measurements to determine the ablation pattern. Conventional corneal topography measurements are typically noisy and can include gaps in the measured field. Wavefront aberrometers and other in vivo ophthalmic measurement devices often face the same problem. The full image derived from such devices can be restored by decomposing available data into Zernike series or any other orthogonal basis functions. However, the accuracy of such decomposition, depending on the measurement noise level and the sample size of available data, needs to be evaluated. Oftentimes, there is a need to optimally combine several arbitrary sets of measurement data together with a *priori* information about the measured field.

For example, corneal or wavefront data acquired by medical diagnostics devices, such as the WaveScan® aberrometer by Abbott Medical Optics Inc. or the Atlas™ corneal topographer by Carl Zeiss Meditec, Inc., are typically converted to a two-dimensional field by known interpolation scheme or through decomposition of available data into Fourier or orthogonal polynomials with subsequent reconstructions of the two-dimensional field from those decompositions. The quality of such reconstructions remains unclear and can be assessed only qualitatively. Any *priori* statistics of the measured field are usually not considered. If several measurements are performed, a simple average of several measurements is typically chosen as a final measurement outcome without consideration of comparative quality of different measurement data.

A thesis by Weaam F. M. Alkhaldi: "Statistical Signal and Image Processing Techniques in Corneal Modeling", 13 July 2010, XP055197659, http://tuprints.ulb.tu-darmstadt.de/2232/2/WeaamAlkhaldi_PhD_Thesis.pdf, deals with corneal topography reconstruction.

In light of the above, it would be desirable to provide improved ophthalmic measurement data assimilation techniques, particularly for refractive correction.

SUMMARY OF THE INVENTION

The present invention provides ophthalmic treatment and diagnostic techniques based on measurement data assimilation of corneal topography and/or wavefront measurements. In one particular embodiment, the data assimilation technique is based on a Kalman-Bucy technique to combine measured corneal elevations with a *priori* information, including mean and covariance of Zernike amplitudes for a measured surface known prior to the measurement. This technique provides a statistically optimal estimate of post-measurement mean and covariance of Zernike *amplitudes.* *A priori* mean and covariance before any measurements can be estimated from an ensemble of available data. Repeated measurements of the same eye can use mean and covariance from the preceeding measurement in combination with the current measurement. In some embodiments of the present invention, systems are provided for treating a tissue of an eye with a laser beam based on an ablation pattern or refractive correction determined from the corneal topography and wavefront measurements assimilated in accordance with the foregoing technique. In some embodiments of the present invention, systems and methods are provided for determining refractive corrections of an eye from corneal topography and wavefront measurements assimilated in accordance with the foregoing technique.

The disclosure comprises a method of treating a cornea of a patient's eye with a laser beam. Angles between a surface of a cornea and a laser beam are mapped over a treatment area. Ablation properties are determined locally across a treatment area in response to mapped angles so as to formulate a treatment plan using local ablation properties. A treatment area is ablated according to the treatment plan to form a desired shape in a surface.

In some embodiments, an angle of a laser beam may be substantially parallel to an optical axis of an eye. A mapped area includes an apex of a cornea and an apex of a cornea is displaced from a center of a pupil of an eye. A desired shape has a center, and a center of a desired shape may be aligned with a center of a pupil of an eye. A virtual shape may be adjusted from a first virtual shape to a second virtual shape. A first virtual shape may represent a depth of material removed from an area to form a desired shape. A second virtual shape may be formed from a first virtual shape in response to the mapped angles. In an embodiment, a depth of a second virtual shape may be greater than a depth of a first virtual shape. In another embodiment, a depth of a second virtual shape may be less than a depth of a first virtual shape. A desired shape may be based at least in part on a result of measurement selected from a group consisting of an aberration measurement of an eye, a refractive measurement of an eye, and a topography measurement of an eye.

In another aspect, the invention comprises a system for treating a cornea of a patient's eye with a laser beam. The system includes a laser emitting a beam of an ablative light energy and at least one processor. At least one processor has a computer program mapping angles between a surface of a cornea and a laser beam. At least one processor determines local ablation properties of a cornea in response to mapped angles. At least one processor has a computer program controlling an ablative treatment in response to local ablation properties. A treatment forms a desired shape in a surface.

In some embodiments, an angle of a laser beam may be substantially parallel to an optical axis of an eye. A mapped area may include an apex of a cornea, and an apex of a cornea may be displaced from a center of a pupil of an eye. A desired shape may have a center, and a center of a desired shape may be aligned with a center of a pupil of an eye. At least one processor having a computer program may include a first virtual shape and a second virtual shape. A first virtual shape may represent a depth of material removed from an area to form a desired shape, and a second virtual shape may be formed from a first virtual shape in response to mapped angles. In an embodiment, a depth of a second virtual shape may be greater than a depth of a first virtual shape. In another embodiment, a depth of a second virtual shape may be less than a depth of a first virtual shape. A desired shape may be based at least in part on a result of measurement selected from a group consisting of an aberration measurement of an eye, a refractive measurement of the eye, and a topography measurement of the eye.

In a further aspect, the invention comprises a system for treating a cornea of an eye with a laser beam. A system includes a laser emitting a beam of an ablative light energy and at least one processor having a computer program. At least one processor determines angles between a curved surface and a laser beam. At least one processor has a computer program controlling an ablative treatment in response to angles between a curved surface and a laser beam. A treatment forms a desired shape in a surface.

In specific embodiments, at least one processor determines local ablation properties of a cornea in response to angles between a curved surface and a laser beam. An angle of a laser beam is substantially parallel to an optical axis of an eye. A mapped area includes an apex of a cornea and an apex of a cornea is displaced from a center of a pupil of an eye. A desired shape has a center, and a center of a desired shape is aligned with a center of a pupil of an eye. At least one processor has a computer program including a first virtual shape and a second virtual shape. A first virtual shape represents a depth of material removed from an area to form a desired shape. A second virtual shape is formed from a first virtual shape in response to mapped angles. In an embodiment, a depth of a second virtual shape is greater than a depth of a first virtual shape. In another embodiment, a depth of a second virtual shape is less than a depth of a first virtual shape. A desired shape is based at least in part on a result of a measurement selected from a group consisting of an aberration measurement of an eye, a refractive measurement of the eye, and a topography measurement of an eye.

The invention is defined in the claims. Other embodiments are exemplary.

BRIEF DESCRIPTION OF THE DRAWINGS

In the drawings, wherein like reference numerals refer to similar components:

FIG. 1 illustrates a laser system ablating a tissue surface in accord with an embodiment of the present invention.

FIG. 1A is a perspective view of a laser ablation system for incorporating the present invention.

FIGS. 2 and 3 schematically illustrate a laser beam delivery system for selectively directing a laser beam onto a corneal tissue in accord with an embodiment of the present invention.

FIG. 4 is a functional block diagram illustrating a control architecture of an ablation system as in FIG. 1A in accord with an embodiment of the present invention.

FIG. 5 is a flow chart schematically illustrating a method for determining a corneal ablation treatment program in accord with an embodiment of the present invention.

FIG. 6 illustrates a laser treatment table in accord with an embodiment the present invention.

FIG. 7 illustrates a surface topography of a cornea in accord with an embodiment of the present invention.

FIG. 8 illustrates local surface angles of a corneal surface topography as in FIG. 7 as surface normal vectors in accord with an embodiment of the present invention.

FIG. 8A illustrates a center of a pupil of an eye in relation to a center of a corneal topography measurement in accord with an embodiment of the present invention.

FIG. 8B illustrates a laser surgery system aligned with a center of an eye in accord with an embodiment of the present invention.

FIG. 9 Illustrates angles of incidence of several rays of a laser beam incident on a surface of a cornea in accord with an embodiment of the present invention.

FIG. 9A illustrates angles of incidence of several parallel rays of a laser beam incident on a surface of a cornea in accord with an embodiment of the present invention.

FIG. 10 illustrates laser beams simultaneously overlapping on a surface of a cornea in accord with an embodiment of the present invention.

FIG. 10A illustrates angles of incidence of simultaneously overlapping rays of laser beams incident on a surface of a cornea in accord with an embodiment of the present invention.

FIG. 11 illustrates an ablation rate of a corneal tissue as related to a fluence of a laser beam applied to a tissue surface.

FIG. 12 illustrates a fraction of a light energy transmitted into a corneal tissue as related to an angle of incidence of a laser beam.

FIG. 13 illustrates a fluence factor as related to an incident angle of a laser beam in accord with an embodiment of the present invention.

FIG. 14 illustrates an ablation rate relative to an ablation rate at normal incidence in accord with an embodiment of the present invention.

FIG. 15 illustrates a desired predetermined ablation shape as a first virtual surface warped to form a second virtual surface in accord with an embodiment of the present invention.

FIG. 16 illustrates a crater of material removed with a single pulse of a laser beam as a first virtual surface warped to form a second virtual surface in accord with an embodiment of the present invention.

FIG. 17 illustrates a laser beam incident upon a corneal surface during a LASIK surgical procedure in accord with an embodiment of the present invention.

FIGS. 18A and 18B are graphs illustrating corneal elevation measurements.

FIGS. 19A and 19B are graphs illustrating deviations of the corneal elevation measurements shown in FIGS. 18A and 18B from an average corneal shape, respectively.

FIG. 20A is a graph illustrating corneal elevation uncertainty for an a *priori* measured corneal shape.

FIG. 20B is a graph illustrating corneal elevation uncertainty for a post-measurement corneal shape.

FIG. 21 is a chart illustrating a *priori* and measured Zernike amplitudes.

FIG. 22A is graph illustrating standard deviation of a corneal elevation field for different eyes.

FIG. 22B is a graph illustrating standard deviation of a corneal elevation field for the same eye.

FIG. 23A is a graph illustrating radial dependence for the standard deviation of corneal elevation shown in FIG. 22A.

FIG. 23B is a graph illustrating radial dependence for the standard deviation of corneal elevation shown in FIG. 22B.

FIG. 24 is a chart illustrating standard deviation of Zernike amplitudes for the corneal elevation fields shown in FIGS.

22A and 22B.

FIG. 25 is a graph illustrating covariance of Zernike amplitudes for corneal elevation.

DETAILED DESCRIPTION

The present invention is particularly useful for enhancing the accuracy and efficacy of laser eye surgical procedures, such as photorefractive keratectomy (PRK), phototherapeutic keratectomy (PTK), laser assisted in situ keratomileusis (LASIK), laser epithelial keratomileusis (LASEK) and the like. Preferably, the present invention can provide enhanced optical accuracy of refractive procedures by improving a corneal ablation of a refractive treatment program. Hence, while the system and methods of the present invention are described primarily in a context of a laser eye surgery system, it should be understood that techniques of the present invention may be adapted for use in alternative eye treatment procedures and systems such as spectacle lenses, intraocular lenses, contact lenses, corneal ring implants, collagenous corneal tissue thermal remodeling, and the like. The techniques of the present invention can be readily adapted for use with existing laser systems, wavefront sensors, corneal topography systems, phoropters and other optical measurement devices as well as treatment planning regimes. By providing a methodology for determining a laser treatment plan that more accurately reflects various characteristics of one or more optical tissue surfaces of the eye, the present invention may facilitate sculpting of the cornea so that treated eyes more accurately result in a desired visual correction.

As used herein an "optical tissue surface" may encompass a theoretical tissue surface derived from an optical measurement of light refraction of an eye (exemplified by wavefront sensor data and manifest refraction data), an actual tissue surface, and/or a tissue surface formed for purposes of treatment (for example, by incising corneal tissues so as to allow a flap of the corneal epithelium to be displaced and expose the underlying stroma during a LASIK procedure).

Systems and methods for measuring a refractive error of an eye such as spherical defocus and cylindrical astigmatism having an axis are well known in the optometric and ophthalmic fields. Examples of measurements of a refractive error of an eye are manifest, cycloplegic, and retinoscopic refraction. U.S. Pat. No. 5,163,934, describes a shape of tissue to be removed from a cornea of an eye to correct a refractive error of an eye. Systems and methods for measuring a corneal topography of an eye are well known in the optometric and ophthalmic fields. For example, U.S. Pat. Nos. 4,761,071, 4,995,716, 5,406,342, 6,396,069, 6,116,738, 4,540,254 and 5,491,524, describe systems and methods for measuring a corneal topography of an eye. Systems and methods for determining an ablation location and shape using corneal topography are described in U.S. Pat. Nos. 6,245,059, 6,129,722 and 5,843,070,

Wavefront sensors will typically measure aberrations and other optical characteristics of an entire optical tissue system. Data from such a wavefront sensor may be used to generate an optical tissue surface from an array of optical gradients. In some instances, an optical tissue surface may be referred to as a wavefront elevation map. An optical tissue surface may not precisely match an actual tissue surface. For example, optical gradients will show effects of aberrations, which are actually located throughout an ocular tissue system. Nonetheless, corrections imposed on an optical tissue surface so as to correct aberrations derived from gradients should correct an optical tissue system. Systems and methods for measuring and correcting aberrations of an optical tissue surface of eye based on wavefront elevation maps are described in U.S. Pat. Nos. 5,777,719, 6,042,012, 6,095,651, 6,199,986, 6,271,914 and 6,217,915,

In correcting an optical tissue surface of an eye, a shape of tissue to be removed is typically determined prior to ablation. A predetermined shape is often the result of a combination of refractive error, wavefront sensor and topography measurements as described above.

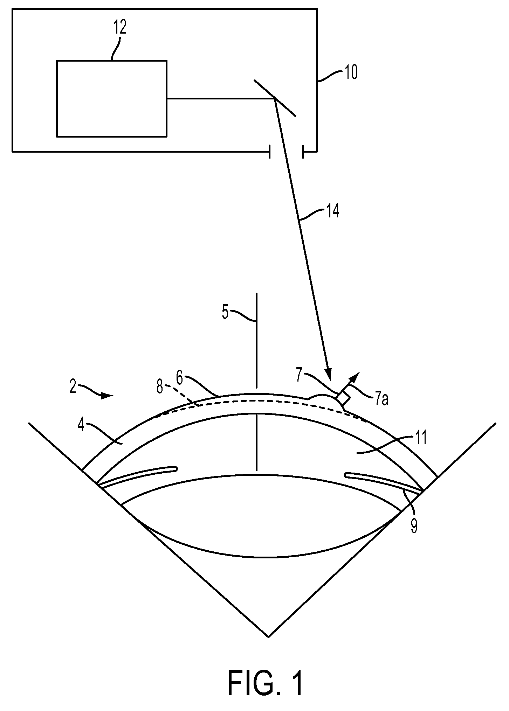

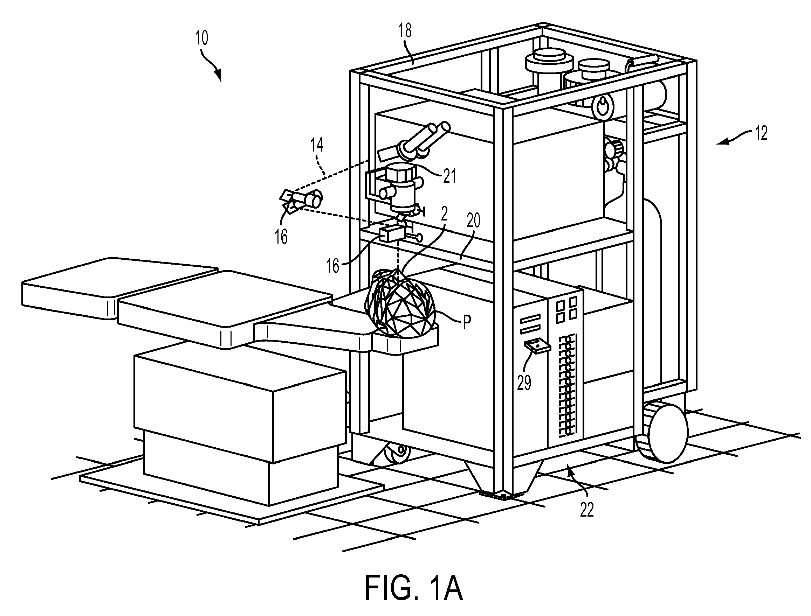

A laser ablating a surface of an eye is illustrated in FIG. 1 in accordance with an embodiment of the invention. An eye 2 is illustrated in cross section as being ablated by a laser system 10 having a laser 12 emitting a beam 14 of an ablative light energy. The eye 2 has a cornea 4 and a pupil 11 formed in an iris 9. The cornea 4 has a surface 6 and a local surface angle 7 associated therewith. In some embodiments, the local surface angle 7 is a surface normal vector 7a, but can be any representation of a local slope of surface 6. The eye 2 has at least one axis, for example an optical axis 5, and the optical axis 5 is preferably aligned with the laser system 10. A desired predetermined shape 8 is formed in the surface 6 with a series of pulses of the laser beam 14 of ablative light energy.

As tissue ablates from surface 6 to form the predetermined shape 8, an amount of tissue ablated with each pulse of laser beam 14 varies with an angle between the surface angle 7 and the laser beam 14. Typically, an amount of tissue removed with each pulse of the laser beam 14 will decrease as the local surface having the angle 7 faces away from the laser beam 14. By determining a local amount of ablation from a local angle between a local surface angle and a local angle of laser beam incident on the local surface, a treatment program will more accurately calculate a distribution pattern of a series of pulses to form the desired predetermined shape 8.

Referring now to FIG. 1A, a laser eye surgery system 10 for incorporating the present invention includes the laser 12 that produces a laser beam 14. Laser delivery optics 16 are in a path of laser beam 14, and the delivery optics 16 direct the laser beam 14 to an eye of a patient P. A delivery optics support structure (not shown here for clarity) extends from a frame 18 supporting the laser 12. An input device 20 is used to align laser system 10 in relation to an eye of a patient P. A microscope 21 is mounted on the delivery optics support structure, the microscope often being used to image a cornea of an eye. In various embodiments, a laser eye surgery system 10 includes at least some portions of a STAR S4 IR® Excimer Laser System available from Abbott Medical Optics Inc..

While the input device 20 is schematically illustrated as a joystick, a variety of input components may be used. Suitable input components may include trackballs, touch screens, or a wide variety of alternative pointing devices. Still further alternative input components include keypads, data transmission mechanisms such as an Ethernet, intranet, Internet, a modem, or the like.

The laser 12 generally comprises an excimer laser, such as an argon-fluoride laser producing pulses of laser light having a wavelength of approximately 193 nm. The pulse of laser light typically has a fixed pulse duration having a full width half maximum (FWHM) of about 15 nanoseconds during a treatment. The laser 12 is preferably designed to provide a feedback stabilized fluence at the patient's eye, delivered via the delivery optics 16. The present invention may also be useful with alternative sources of ultraviolet or infrared radiation, particularly those adapted to controllably ablate a corneal tissue without causing significant damage to adjacent and/or underlying tissues of the eye. The laser system may include, but is not limited to, excimer lasers such as argon-fluoride excimer lasers (producing laser energy with a wavelength of about 193 nm), solid state lasers, including frequency multiplied solid state lasers such as flash-lamp and diode pumped solid state lasers. Exemplary solid state lasers include UV solid state lasers (approximately 193-215 nm) such as those described in U.S. Pat. Nos. 5,144,630 and 5,742,626, Borsuztky et al., "Tunable UV Radiation at Short Wavelengths (188-240 nm) Generated by Sum Frequency Mixing in Lithium Borate", Appl. Phys. 61:529-532 (1995), and the like. Laser energy may comprise a beam formed as a series of discreet laser pulses. A variety of alternative lasers might also be used. Hence, although an excimer laser is the illustrative source of an ablating beam, other lasers may be used in the present invention.

The laser 12 and delivery optics 16 generally direct the laser beam 14 to an eye of patient P under direction of a processor 22. The processor 22 often selectively adjusts the laser beam 14 to expose portions of the cornea to pulses of laser energy so as to effect a predetermined sculpting of a cornea and alter refractive characteristics of an eye. In many embodiments, both the laser 14 and laser delivery opticals 16 are under computer control of the processor 22 to effect a desired laser sculpting process, with the processor 22 effecting (and optionally modifying) a pattern of laser pulses. A pattern of pulses may by summarized in a treatment table listing of machine readable data of a tangible storage media 29. The treatment table can be adjusted according to feedback input into the processor 22 from an automated image analysis system (e.g., manually input into processor 22 by a system operator) in response to feedback data provided from an ablation monitoring system feedback system. Such feedback might be provided by integrating a wavefront measurement system described below with the laser treatment system 10, and the processor 22 may continue and/or terminate a sculpting treatment in response to feedback, and may also optionally modify a planned sculpting based at least in part on feedback.

The laser beam 14 can be adjusted to produce a desired sculpting using a variety of alternative mechanisms and may be selectively limited using one or more variable apertures. An exemplary variable aperture system having a variable iris and a variable width slit is described in U.S. Pat. No. 5,713,892, A laser beam may also be tailored by varying a size and offset of a laser spot from an axis of an eye, as described in U.S. Pat. No. 5,683,379, and as also described in co-pending U.S. patent application Ser. Nos. 08/968,380, filed Nov. 12, 1997; Ser. No. and 09/274,999 filed Mar. 22, 1999.

Still further alternatives are possible, including scanning a laser beam over a surface of an eye and controlling a number of pulses and/or dwell time at each location, as described, for example, by U.S. Pat. No. 4,665,913 using masks in an optical path of the laser beam 14 which ablate to vary a profile of a beam incident on a cornea, as described in U.S. patent application Ser. No. 08/468,898, filed Jun. 6, 1995; hybrid profile-scanning systems in which a variable size beam (typically controlled by a variable width slit and/or variable diameter iris diaphragm) is scanned across the cornea as described in U.S. Pat. Nos. 6,319,247; 6,280,435; and 6,203,539, or the like. The computer programs and control methodology for these laser pattern tailoring techniques are well described in the patent literature.

Additional components and subsystems may be included with the laser system 10, as should be understood by those of skill in the art. For example, spatial and/or temporal integrators may be included to control the distribution of energy within the laser beam, as described in U.S. Pat. Nos. 5,646,791 and 5,912,779, An ablation effluent evacuator/filter, and other ancillary components of the laser surgery system that are not necessary to an understanding of the invention, and which may be optionally employed, need not be described in detail for an understanding of the present invention.

The processor 22 comprises (or interfaces with) a conventional PC system including standard user interface devices such as a keyboard, a display monitor, and the like. The processor 22 typically includes an input device such as a magnetic or optical disk drive, an internet connection, or the like. Such input devices are often used to download a computer executable code from the storage media 29 embodying any methods of the present invention. The storage media 29 may comprise a floppy disk, an optical disk, a data tape, a volatile or non-volatile memory, or the like, and the processor 22 will include memory boards and other standard components of modern computer systems for storing and executing a computer program code. The storage media 29 may optionally embody wavefront sensor data, wavefront gradients, a wavefront elevation map, a treatment map, a corneal topography map, a measurement of a refraction of an eye, and an ablation table.

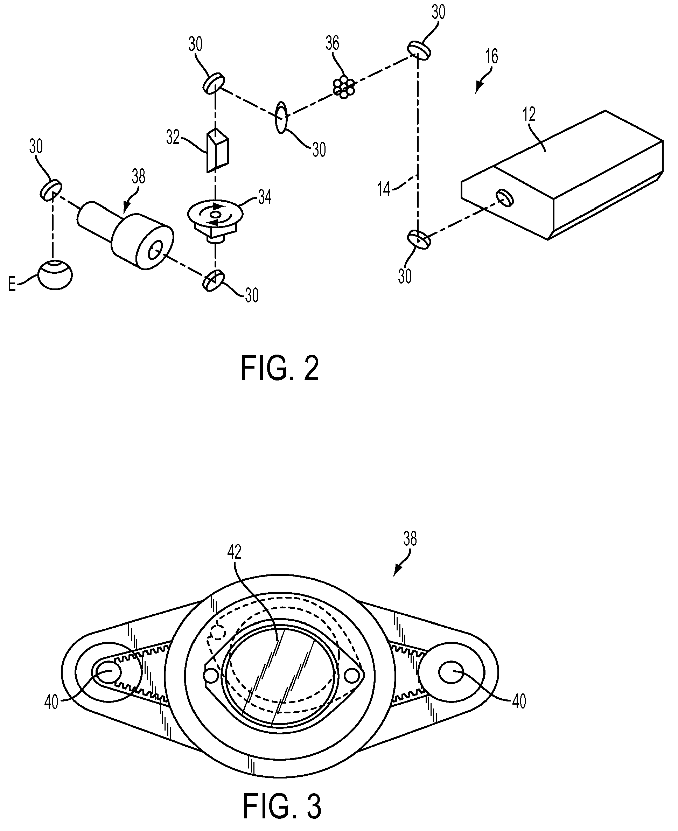

Referring now to FIG. 2, the laser beam delivery system 16 for directing the laser beam 14 at the eye 2 includes a number of mirrors 30, as well as one or more temporal integrators 32 that may adjust (or otherwise tailor) an energy distribution across a laser beam. The laser 12 often comprises an excimer laser as described above.

In an exemplary embodiment, a variable aperture 34 changes a diameter and/or slot width to profile laser beam 14, ideally including both a variable diameter iris and a variable width slot. A prism 36 separates the laser beam 14 into a plurality of beamlets, which may partially overlap on the eye 2 to smooth edges of an ablation or "crater" formed from each pulse of a laser beam. Referring now to FIGS. 2 and 3, an offset module 38 includes motors 40 which vary an angular offset of an offset lens 42, and which also change a radial orientation of an offset. Hence, the offset module 38 can selectively direct the laser beam 14 at a desired lateral region of a cornea. A structure and method for using embodiments of the laser beam delivery system 16 and the offset module 38 are more fully described in U.S. Pat. Nos. 6,331,177; 6,203,539; 5,912,775; and 5,646,791.

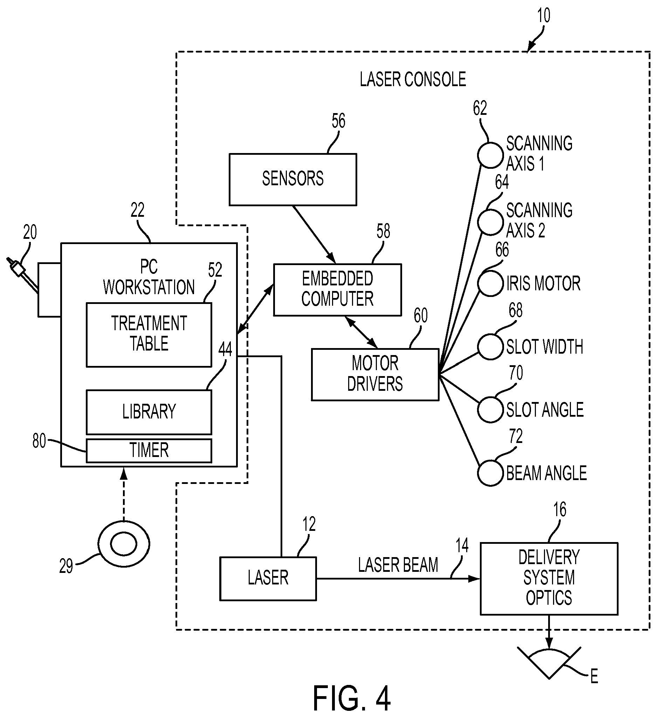

Referring now to FIG. 4, a control system of the laser system 10 is schematically illustrated according to principles of the present invention. The processor 22 enables precise control of the laser system 10 to sculpt a surface shape according to a laser treatment table 52. The processor 22, generally comprising a PC workstation, makes use of a computer program stored on the tangible media 29 to generate the treatment table 52. The processor 22 includes a library 44 of treatments, such as described in U.S. Pat. No. 6,245,059, An embedded computer 58 within the laser system 10 is in electronic communication with the PC workstation. Alternatively, a PC workstation may be embedded in the laser system 10 and include an embedded processor card in communication with a PC workstation for directing an ophthalmic surgery.

The embedded computer 58 is in electronic communication with a plurality of sensors 56 and a plurality of motor drivers 60. The motor drivers 60 are coupled to the embedded computer 58 to vary a position and configuration of many of the optical components of the delivery optics 16 according to the treatment table 52. For example, first and second scanning axes 62, 64 control a position of an offset lens to move several laser beamlets over a tissue surface of the cornea. An iris motor 66 controls an overall diameter of a beam, and in some cases, a length of light transmitted through a variable width slot. Similarly, a slot width driver 68 controls a width of a variable slot. A slot angle driver 70 controls rotation of a slot about its axis. A beam angle driver 72 controls beam rotation as effected by a temporal integrator as described above. A timer 80 controls a time interval between pulses of a laser treatment. The timer 80 measures a time interval from a previous pulse and generates an interrupt after a predetermined time interval has elapsed. The processor 22 issues a command for the laser 12 to generate a pulse of the laser beam 14 after various optical elements have been positioned to create a desired crater on the eye 2 and after a measured time interval has elapsed. The treatment table 52 comprises a listing of all desired craters to be combined so as to effect a treatment therapy.

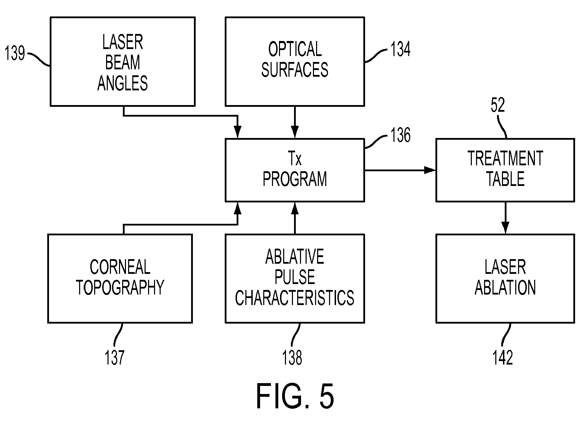

A flow chart schematically illustrating a method for determining a corneal ablation treatment plan is illustrated in FIG. 5 in accordance with an embodiment of the present invention. A treatment calculation program 136 uses properties of an optical tissue surface 134, corneal topography 137, ablative pulse characteristics 138, and laser beam angles 139 to determine a treatment plan listed in the treatment table 52. The optical tissue surface 134 includes information related to optical aberrations of the eye as described above. The corneal topography 137 includes a measured shape of at least one surface of the cornea, preferably a front or anterior surface as described above, and the corneal topography 137 preferably includes information locating a center of a pupil in relation to mapped corneal topography coordinates. The ablative pulse characteristics 138 include information describing a shape of tissue, or "crater" removed with a pulse of a laser beam. The local laser beam angles 139 include information describing several local angles of several rays for at least one laser beam incident on several locations of the cornea in relation to a reference axis, for example an optical axis of an eye as described above.

The treatment calculation program 136 combines information from the optical tissue surface 134 with corneal topography 137 to determine a desired shape of tissue to be removed from the surface 6 of the cornea 4 to form the desired shape 8 in the surface 6 in one embodiment. In another embodiment, a desired shape of tissue to be removed from the surface 6 may be calculated from an optical tissue surface, for example from a wavefront elevation map, without using corneal topography information. In another embodiment, the desired shape of tissue to be removed from the surface 6 may be calculated from the corneal topography information without using the wavefront information. The desired shape of tissue removed is preferably determined from athe optical tissue surface 134 so as to remove regular (spherical and/or cylindrical) and irregular errors of optical tissues as described above. Alternatively, the desired shape of tissue to be removed may be determined so as to modify the optical tissue surface 134 and leave controlled amounts of aberration, for example controlled amounts of aberrations correcting presbyopia or extending a depth of focus.

By combining in a treatment plan an optical tissue surface and the ablative laser pulse characteristics 138 of a particular laser system, the treatment table 52 of ablation pulse locations, sizes, shapes, and/or numbers can be developed. An exemplary method and system for preparing such an ablation table is described in U.S. Pat. Nos. 6,673,062 and 7,008,415, Sorting of individual pulses to avoid localized heating, minimize irregular ablations if the treatment program is interrupted, and the like may optionally optimize the treatment table 52. Preferably, a series of pulses applied to an eye are listed in a treatment table and sorted to initially apply pulses having a small cross sectional dimension followed by pulses having a larger cross sectional dimension. Alternatively, a treatment table may be sorted to apply large diameter pulses to an eye initially followed by smaller diameter pulses, and an order of pulses may provide pulses having a random size distribution. An eye can then be treated by laser ablation 142 according to the treatment table 52.

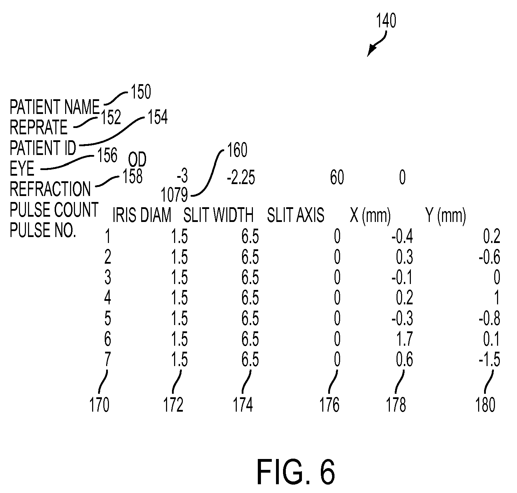

Referring now to FIG. 6, several listings from an exemplary laser treatment table 140 are illustrated. A Patient Name 150, patient identification number (Patient ID) 154, and treated Eye 156 are listed in a treatment table 140. A repetition rate (reprate) 152 is also listed. A refraction 158 having a sphere of -3 D, a cylinder of -2.25D, an axis of 60 degrees and a vertex distance of 0 mm is listed in FIG. 6. A pulse count 160 as listed in FIG. 6 illustrates a total number of 1079 pulses applied to an eye during a treatment. Additional fields of the treatment table 140 are pulse number 170, iris diameter 172, slit width 174, slit axis 176, X coordinate 178, and Y coordinate 180.

For each pulse of the treatment table 140, a pulse number 170, iris diameter 172, slit width 174, slit axis 176, X coordinate 178 and Y coordinate 180 are listed. The X coordinate 178 and Y coordinate 180 list X and Y coordinates of a center of each pulse on a cornea relative to a treatment center during a treatment. An iris diameter field 172 lists a dimension across a circular iris diaphragm opening as projected onto an eye in millimeters for each pulse during treatment as described above. A slit width field 174 and a slit axis field 176 list a dimension across and an angle of a variable width slot opening as projected onto an eye as described above. A laser treatment table for scanning a variable width slot is described in U.S. Pat. No. 6,203,539,

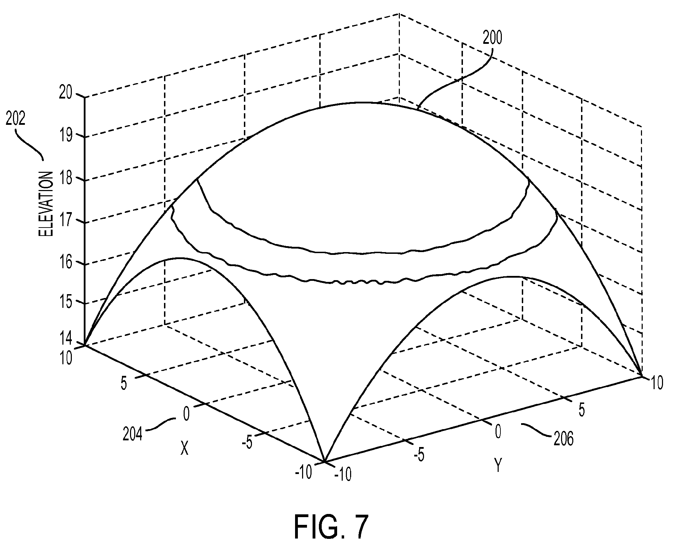

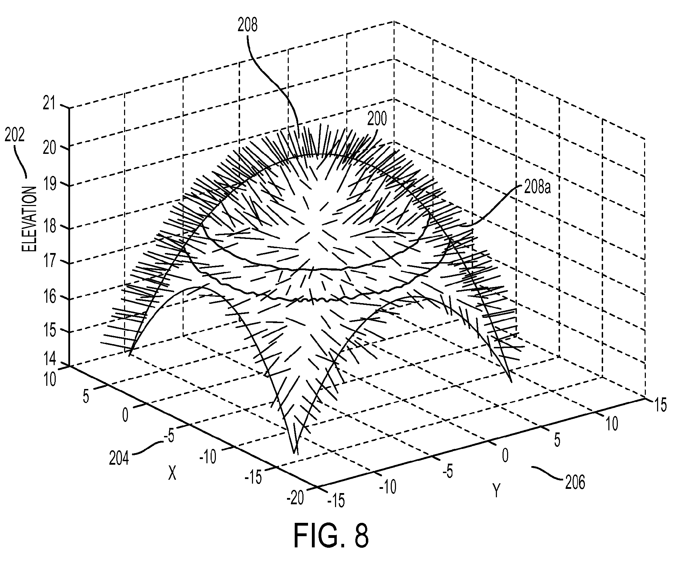

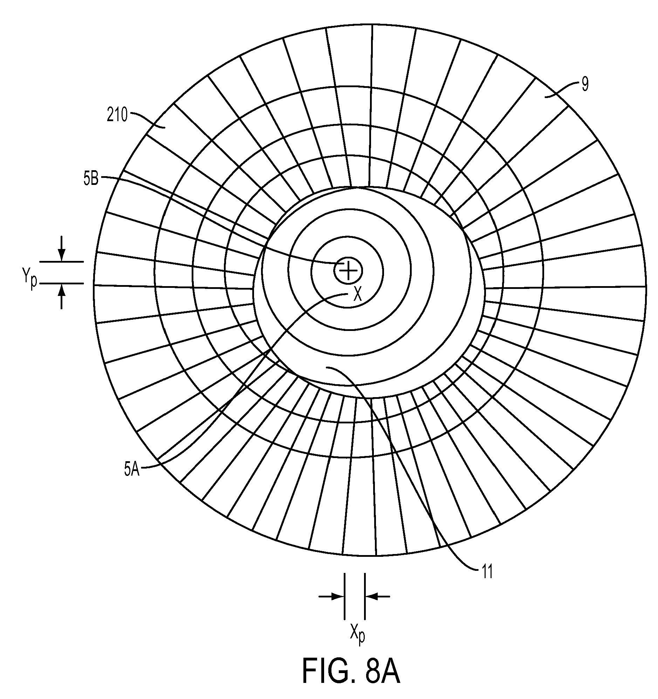

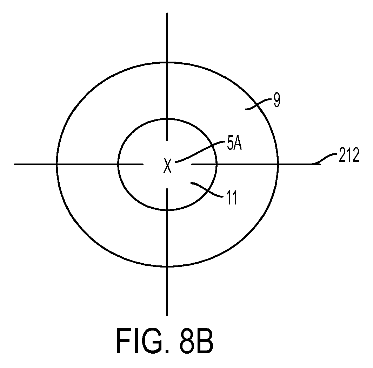

A map 200 of corneal surface elevation is illustrated in FIG. 7. The map 200 has an elevation 202 along a first dimension X 204 and a second dimension Y 206. For the map 200 of corneal surface elevation, several local surface angles are determined over a map 200 of a corneal surface as shown in FIG. 8. Several local surface angles are represented as several local surface normal vectors 208. An individual surface normal vector 208a is illustrated. The map 200 of corneal surface elevation 202 is expressed as a function Z(x,y) of the first dimension X 204 and the second dimension Y 206. Based on an elevation map, a surface normal vector 210 can be computed from Z(x,y) as:

$$N(x,y)=(Z_u \times Z_v)$$

where $Z_u$ and $Z_v$ are partial derivatives of the surface at point Z(x,y). A surface normal vector is preferably normalized to have a magnitude of 1. A normalized surface normal vector is expressed as $n(x,y)=N(x,y)/\|N\|$.

A measurement of a corneal topography of an eye and a pupil of an eye are illustrated in FIG. 8A. The pupil 11 is formed in the iris 9. Several rings 210 of light are reflected from a surface of a cornea during a topography measurement of the cornea. In this embodiment, a center of a topography measurement is near an apex of a cornea 5B. A center of a pupil 5A is illustrated as displaced from an apex of the cornea 5B. A center of a topography measurement is displaced from a center of a pupil by distances $X_p$ and $Y_p$ along first and second dimensions X and Y, respectively. Several commercially available corneal topography systems measure a corneal topography of an eye and a center of a pupil of an eye. For example, a Humphrey® Atlas™ Corneal Topography System is available from ZEISS HUMPHREY SYSTEMS.

An alignment of an eye with the laser system 10 as described above is illustrated in FIG. 8B. The pupil 11 is formed in the iris 9. A reticule 212 is aligned with the center 5A of the pupil 11. In some embodiments, the system 10 may be aligned with any center of an eye, for example a center of a reflected image, such as a first Purkinje image, a center of a dilated pupil, and a center of a limbus.

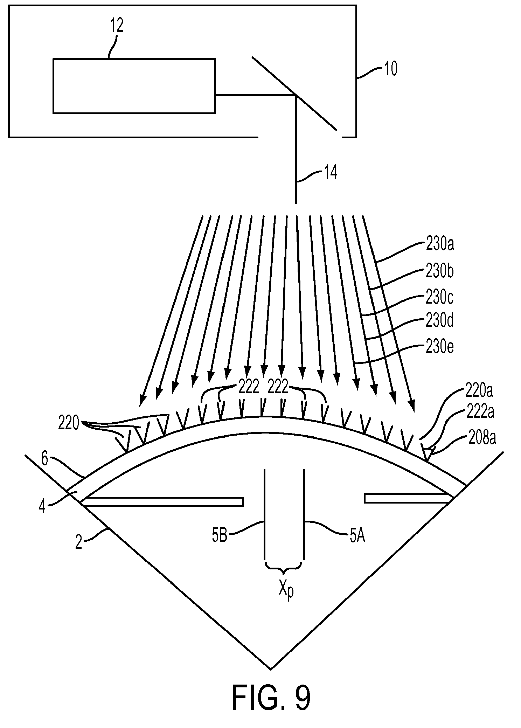

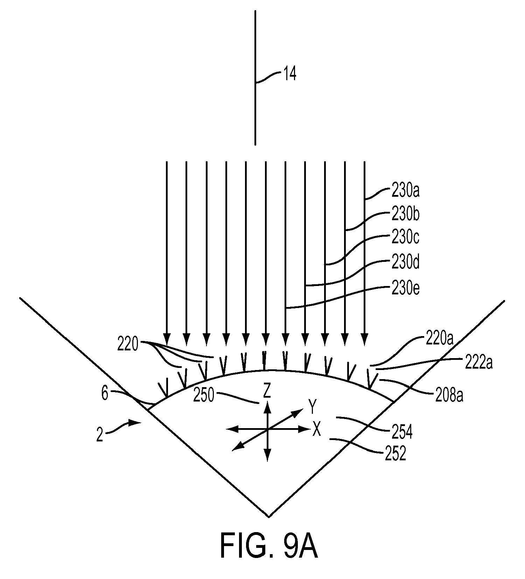

A surgery and an optical tissue surface measurement are centered about a pupil of an eye as illustrated in FIG. 9. The center of the pupil 5A of the eye has an associated line of sight passing through the cornea of the eye as a patient looks at a fixation target. A line of sight is also referred to as a chief ray. A wavefront measurement of an eye is commonly centered about a pupil of an eye. A topography system generally has a central coordinate reference near an apex of the cornea 5B. A topography system may use any reference point as a coordinate center. Surface normal vectors are desirably calculated in relation to a center of the pupil. A pupil center in a topography system measurement reference system may be expressed as $(X_p, Y_p)$. A separation distance $X_p$ from the apex of the cornea 5B to the center of the pupil 5A is illustrated in FIG. 9. In a coordinate system centered about the pupil, surface normal vectors are represented as N(x',y') where

$$x'=x- X_p$$

and

$$y' = y - Y_p.$$

A pupil-centered vector field N(x',y') is used to derive a local incident angle map $\Theta(x',y')$ as a function of local position on a surface of an eye. The local incident angle map $\Theta(x',y')$ describes a local angle at which a laser beam strikes a surface.

As illustrated in FIG. 9, the laser system 10 has the laser 12 that emits the laser beam 14 as described above. Several rays 230a to 230e of the laser beam 14 are illustrated. A local angle of the ray 230a of the laser beam 14 incident on the cornea is illustrated as a ray normal vector 220a. The ray normal vector 220a representing an angle of a laser beam is preferably a normalized vector (i.e. has a magnitude of one). Several ray normal vectors 220 of a map of ray normal vectors are illustrated in FIG. 9. Mathematically, a map of ray normal vectors is expressed as r(x',y'). A map of ray normal vectors is readily calculated for any laser system with a ray tracing program. A map of ray normal vectors is calculated in relation to an optical axis of a laser system 10 that is aligned with a center, preferably the pupil center 5A, during surgery.

The local incident angle map $\Theta(x',y')$ describes a local angle between a surface normal vector and a local angle of a laser beam incident on an eye. The local incident angle map $\Theta(x',y')$ is used to determine local ablation properties of a tissue. For each of several local incident angles, a local tissue ablation property is determined. A treatment table is generated based at least in part on a local ablation property.

Several local incident angles 222 of the local incident angle map $\Theta(x',y')$ are illustrated in FIG. 9. A local incident angle 222a between a local surface normal vector 208a and a local ray normal vector 220a of a laser beam is illustrated. The local angle of incidence 222a is related to a dot product projection of the local ray normal vector 220a and the surface normal vector 208a. The local incident angle map $\Theta(x',y')$ is calculated from a dot product projection of surface normal vectors 208 and several ray normal vectors 220 as

$$\Theta(x',y') = \cos^{-1[r(x',y')n(x',y')]},$$

In an embodiment illustrated in FIG. 9A, several rays 230a to 230e of the laser beam 14 are incident on the surface 6 of the cornea of the eye 2. Several rays 230a to 230e of the laser beam 14 are parallel. A Z axis 250 is perpendicular to a plane of X and Y coordinate references 252, 254 respectively. The Z axis 250 is parallel to rays 230a to 230e. In this embodiment, the Z axis 250 is parallel to several local ray normal vectors 220 and the ray normal vector 220a. The local angle of incidence 222a is related to a dot product projection of the local ray normal vector 220a and the surface normal vector 208a as described above. As ray normal vectors 220 are parallel to the Z axis 250, the local angle of incidence is related to a dot product projection of Z axis 250 and a surface normal vector. the local incident angle map $\Theta(x',y')$ may be calculated as

$$\Theta(x',y') = \cos^{-1(N_{z(x',y')}/\|N\|)}$$

where $N_z$ is the z-component of the surface normal and $\|N\|$ is the magnitude of the surface normal vector.

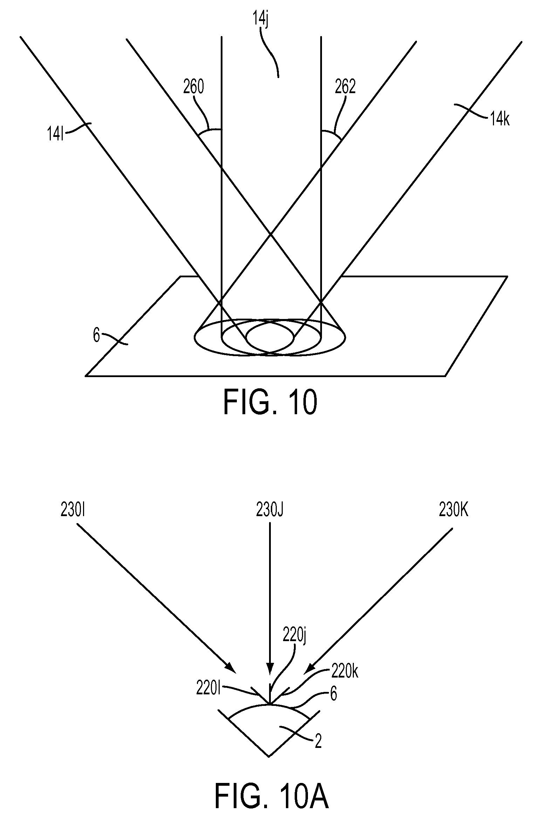

In an embodiment illustrated in FIG. 10, the laser beam 14 as described above is divided into several smaller laser beams, for example beams 14I, 14J and 14K. The laser beams 14I, 14J and 14K overlap and are incident on the surface 6 of the cornea. The laser beams 14I and 14I are separated by an angle 260. The laser beams 14J and 14K are separated by an angle 262. Systems and methods for multiple beam laser sculpting are described in U.S. Pat. No. 6,331,177.

The laser beams 14I, 14J and 14K include rays 230I, 230J and 230K incident on a common location on the surface 6 of the cornea of the eye 2, as illustrated in FIG. 10A. The ray normal vectors 220I, 220J and 220K describe an angular orientation of each of the beams 14I, 14J and 14K, respectively at a common location on the surface 6. An angle of incidence between each ray and a surface normal vector is calculated as described above. In some embodiments, the angles 260 and 262 are small and the ray normal vectors 220I, 220J and 220K are assumed to be accurately represented by a single ray normal vector, for example ray normal vector 220J.

A local angle of incidence of a laser beam on a corneal surface is used to determine local ablation properties. An amount of light locally transmitted into a tissue is related to the angle of incidence of the laser beam. Several factors contribute to the amount of light transmitted into the tissue. Reflection of light energy from a surface is one such factor. Another factor is an effective increase in a size of surface area irradiated by the laser beam.

An effective fluence of a light beam applied on a surface changes with an angle of incidence of a light beam.

A change in an applied fluence with a change in an angle of incidence is referred to as a cosine effect. A beam incident on a surface illuminates an increased area as an angle of incidence increases. For a fixed amount of energy along a cross sectional dimension of a laser beam, an increase in an illuminated area will decrease an amount of energy per unit area applied to a tissue. An effective fluence applied to a surface changes as a cosine of an angle of incidence. For example, a laser beam having a cross sectional diameter of 1 mm and a fluence of 160 mJ/cm$^2$ will irradiate a 1 mm cross sectional diameter of tissue with a fluence of 160 mJ/ cm$^2$ when an angle of incidence is 0. However, a laser beam having a cross sectional diameter of 1 mm and oriented at 45 degrees to a surface will irradiate a cross section of tissue having a length of 1.4 mm along a first dimension and a length of 1 mm along a second dimension. An effective fluence applied to a surface will decrease to 110 mJ/ cm$^2$.

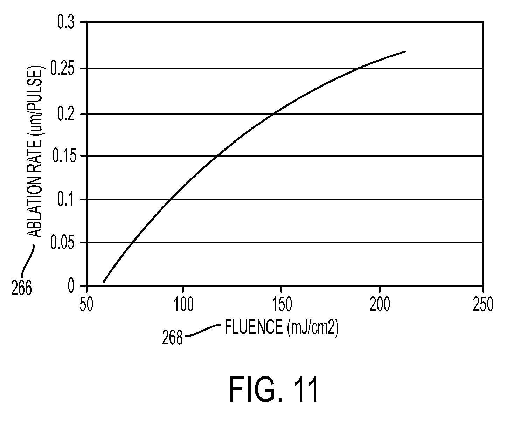

As illustrated in FIG. 11, an amount of tissue ablated with a pulse of a laser beam depends at least in part on an amount of energy per unit area applied to a tissue. An ablation rate 266 changes with a fluence 268 of light energy applied to an eye with a pulse of a laser beam applied. At a fluence 268 of about 160 mJ/ cm$^2$, an ablation rate 266 is illustrated as about 0.23 um per pulse for a laser beam at normal incidence. At a fluence 268 of 110 mJ/ cm$^2$, an ablation rate 266 is illustrated as about 0.11 um per pulse for a laser beam at normal incidence. Systems and methods for measuring tissue ablation rates are known, and alternate embodiments may use a different ablation rate 266 for a similar amount of applied fluence 268.

Amounts of light energy reflected from a surface and transmitted through a surface into a tissue change with a change in an angle of incidence of a light beam. An amount of light energy transmitted into a tissue is calculated with Fresnel formulae. These formulae are known, and use an index of refraction and an angle of incidence to determine an amount of light energy penetrating into a tissue. For an excimer laser as described above polarization is random. In alternate embodiments a laser beam is polarized. A fraction of light energy transmitted into a tissue is determined by a transmissivity expressed as

$$T(\Theta_i) = \{[(\sin 2\Theta_i \sin 2\Theta_t)/(\sin^2(\Theta_i + \Theta_t)\cos^2(\Theta_i - \Theta_t))] +$$

$$[(\sin 2\Theta_i \sin 2\Theta_t)/(\sin^2(\Theta_i + \Theta_t)]\}/2$$

for a randomly polarized light beam, where $\Theta_i$ is an angle of incidence of a light beam and $\Theta_t$ is a transmitted angle of light beam. The angle of incidence $\Theta_i$ of a light beam is related to the transmitted angle $\Theta_t$ by Snell's law. For corneal tissue, an index of refraction is about 1.377. The transmitted angle $\Theta_t$ calculated from Snell's law is expressed as:

$$\sin \Theta_t = \sin \Theta_i / 1.377,$$

where $\Theta_i$ is the angle of incidence of a light ray.

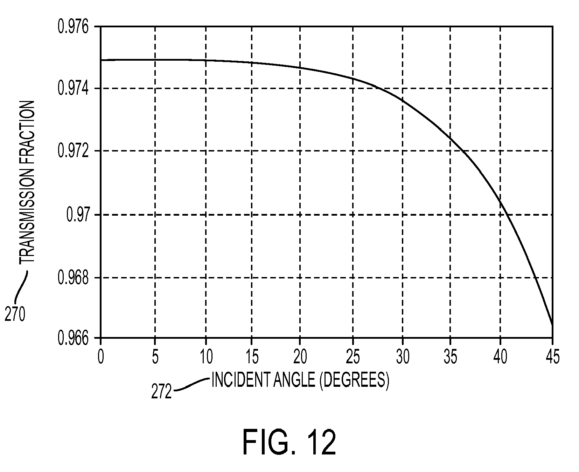

A fraction 270 of energy transmitted into the corneal tissue is illustrated in FIG. 12. The fraction 270 of energy transmitted into the tissue changes with an angle of incidence 272. For an angle of incidence 272 of 0, the fraction 270 of light energy transmitted into a tissue is illustrated as 0.975, or about 98%. For an angle of incidence 272 of 45 degrees, a fraction 270 of energy transmitted is illustrated as 0.966, or about 97%.

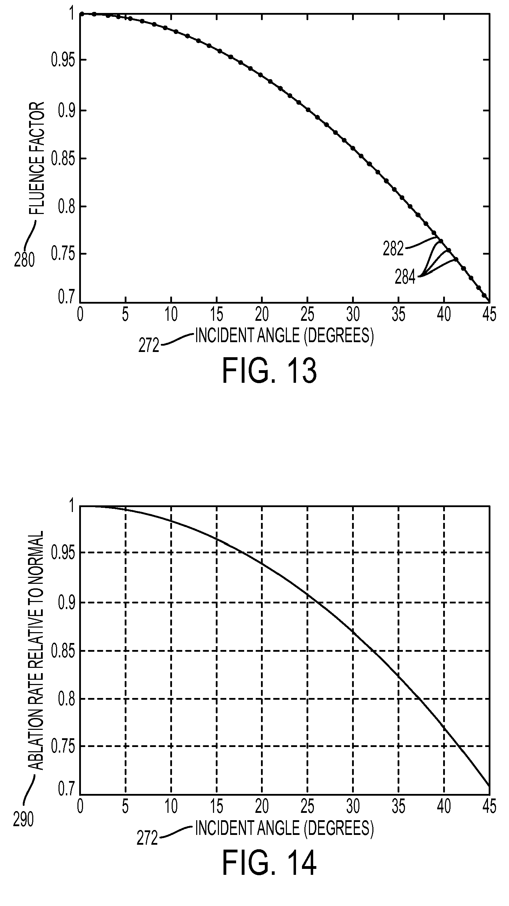

A fluence factor 280 is determined for the angle of incidence 272 as illustrated in FIG. 13. The fluence factor is used to determine an applied local tissue fluence of a laser beam. The fluence factor 280 is a fraction of cross sectional beam energy transmitted through a tissue surface and varies with the incident angle 272. A fluence factor 280 includes a "cosine effect" and a transmission fraction 270 as described above. The fluence factor 280, including both a cosine projection and a transmission fraction 270, is illustrated with several dots 284. In some embodiments, a fluence factor may include a cosine projection of a beam onto a surface and assume reflectance to be uniform across a mapped cornea, as illustrated with a solid line 282. Local tissue fluence is determined at a location by multiplying a fluence factor and a fluence of a laser beam. For a laser beam having a cross-sectional fluence of 160 mJ/cm$^2$ at normal incidence to a surface and a fluence factor of 0.9 at 25 degrees, a tissue fluence is a product of 0.9 and 160 equaling 144 mJ/ cm$^2$. Other embodiments may use a laser beam having a Gaussian energy intensity profile distribution. The local fluence may be calculated by multiplying a fluence factor by a local energy intensity at normal incidence.

For a local angle of incidence of a laser beam, a local fluence transmitted into a tissue is determined. A local tissue ablation rate is determined from the local fluence transmitted into the tissue using a tissue ablation rate as related to fluence applied at normal incidence as described above.

An ablation rate relative to ablation at normal incidence 290 is illustrated in FIG. 14 and varies with an incident angle 272. In an embodiment using a fluence factor, a laser beam fluence and tissue ablation rate as described above, a local tissue ablation rate relative to the tissue ablation rate at normal incidence may be determined. This local tissue ablation property may be used to adjust a laser beam treatment. In some embodiments, the local tissue ablation rate relative to ablation at normal incidence may be accurately determined by a cosine function of an angle of incidence, for example at small angles of incidence.

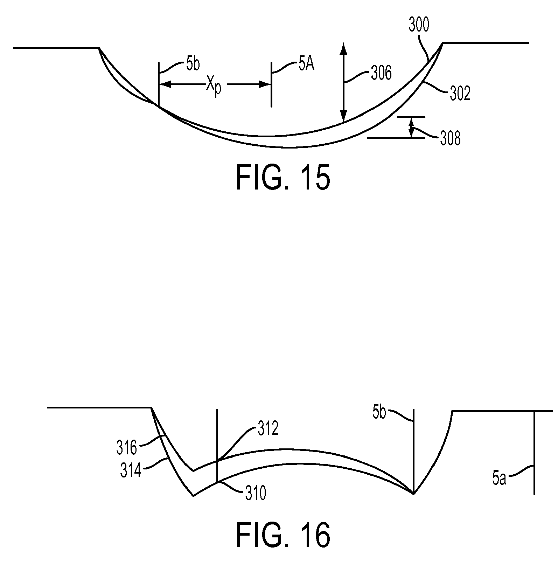

In one embodiment, a predetermined intended ablation shape of tissue removed from a corneal tissue is adjusted to compensate for local ablation properties as illustrated in FIG. 15. In some embodiments, an adjustment of a virtual ablation shape from a first virtual shape to a second virtual shape may be referred to as warping of an ablation target. A predetermined shape of ablation is stored in a memory of a processor as a first virtual shape 300. A local incident angle map is used to determine a map of local ablation properties, for example a map of local ablation rate relative to an ablation rate at normal incidence as described above. A first virtual shape 300 is adjusted by dividing a depth of a first virtual shape 300 by an amount of relative ablation to form a second virtual shape 302. For example, the first virtual shape 300 has a depth 306 of ablation of 10 um at a location. A map of local ablation properties determines relative ablation to be 0.9 locally. The second virtual shape 302 has a local depth of ablation of 11 um that has increased by an amount 308 of 1 um. A treatment plan is determined from the second virtual shape 302 and listed in a treatment table as described above. As a series of pulses is applied to an eye, a shape of ablated tissue matches the first virtual shape 300.

In another embodiment, a simulated shape of material removed with each pulse of a laser beam is adjusted based on local ablation properties as illustrated in FIG. 16. At least one crater of material removed with a single pulse of a laser beam is stored in a memory of a processor as a first virtual surface 314. Systems and methods for determining shapes of tissue removed with a laser beam are described in U.S. Pat. Nos. 6,315,413 and 6,302,876. During a treatment, a final ablated shape of material removed from a surface is a summation of individual craters of tissue removed with each pulse of a series of laser beam pulses. To determine a simulated shape of an ablation, each simulated crater of tissue removed in a series of pulses is adjusted by local ablation properties. As illustrated in FIG. 16, a crater described by a first virtual surface 314 is adjusted using local ablation properties to form a second virtual surface 316. The second virtual surface 316 illustrates a crater of material removed as adjusted based on local ablation properties. A center of a pupil is illustrated at 5A as described above. As illustrated for a treatment centered about a pupil, the first and second virtual surfaces 314 and 316 respectively are displaced from a treatment center as may occur during a scanning treatment.

Preferably, a local fluence of light energy transmitted into a tissue is determined and a local depth of ablation determined as described above. In some embodiments, a depth of ablation may be adjusted by a factor such as an ablation rate relative to an ablation rate at normal incidence as described above. A depth of ablation 310 at a location of the first virtual surface 314 is decreased to a second depth of ablation 312 in the second virtual surface 316 as adjusted based on local ablation properties. A center of a cornea at normal incidence to a laser beam ray is illustrated at 5B as described above. At normal incidence, the depth of the first virtual surface 314 matches the depth of the second virtual surface 316. To determine a predetermined shape of tissue removed by a series of laser beam pulses, several craters are adjusted based on local ablation properties and combined to determine a total shape of material removed. Each crater of a treatment is adjusted based on local ablation properties and a treatment plan is calculated and listed as treatment table as described above.

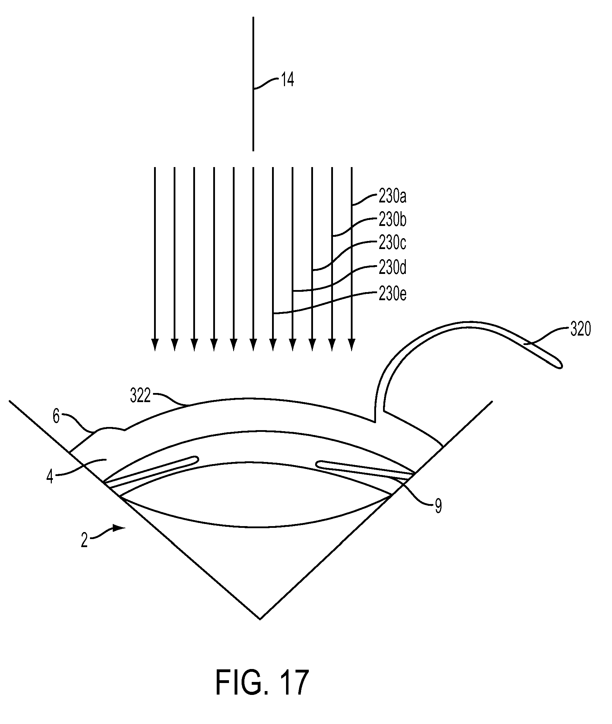

In one embodiment, a LASIK surgical eye procedure is performed on an eye as illustrated in FIG. 17. The eye 2 has a cornea 4. Several rays 230A-230E of the laser beam 14 are incident on a surface of a cornea as described above. Several local angles of incidence of rays of laser beam 14 are determined as described above. Local tissue ablation properties are determined at least in part in response to local incident angles as described above. A flap of corneal tissue 320 is resected from the cornea 4, exposing a bed of stromal tissue 322. In a preferred embodiment, several local angles of incidence are determined before a flap of corneal tissue 320 is resected. In some embodiments, several local angles of incidence and local tissue ablation properties may be determined after a flap of corneal tissue 320 is resected. A laser beam treatment forms a desired ablation shape in the cornea 4 as described above. After ablation, the flap 320 is repositioned over a bed of stromal tissue 322.

**Measurement Data Assimilation**

In a preferred embodiment of the present invention, measurement data assimilation is based on a Kalman-Bucy technique for corneal topography and/or wavefront measurements to provide a statistically optimal estimate of a measured field and a measure of the quality of the measured field. This method combines measured corneal elevations with a *priori* information, including mean and covariance of Zernike amplitudes for a measured surface known prior to the *measurement. A priory* mean and covariance before any measurements can be estimated from available data for a general population. This technique yields a statistically optimal estimate of Zernike amplitudes that represent weighted averages of prior estimates and measured values. Measured values that deviate farther from a *priori* corneal shape can be included with smaller weighting. This provides the technique with robustness for measurement outliers. The data assimilation also yields an estimate for a post-measurement covariance matrix of Zernike amplitudes. Variances of Zernike amplitudes provide a measure of the measurement quality.

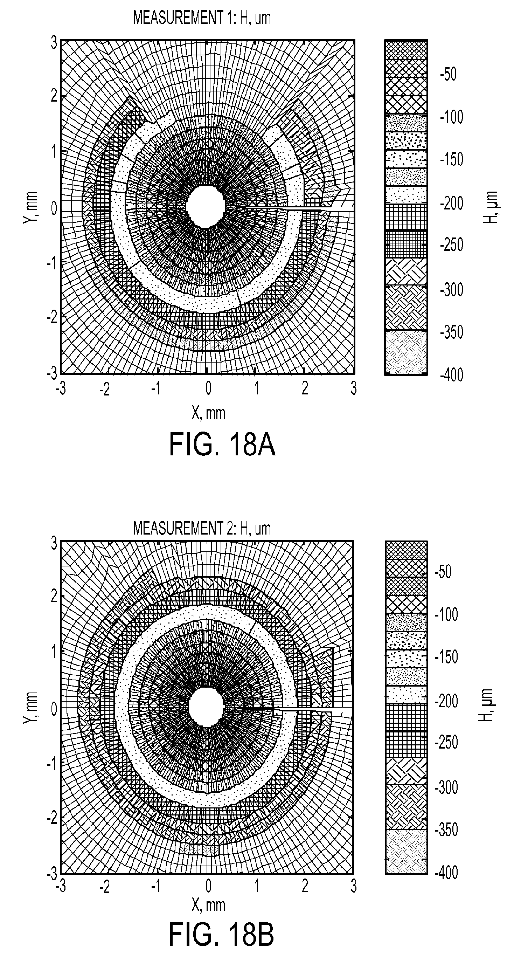

With respect to corneal topography, every topography measurement yields a two-dimension field of corneal elevations and typically contains some gaps and inaccuracies. FIGS. 18A and 18B are graphs illustrating corneal elevation measurements of an eye using a Humphrey® Atlas™ Corneal Topography System from ZEISS HUMPHREY SYSTEMS.

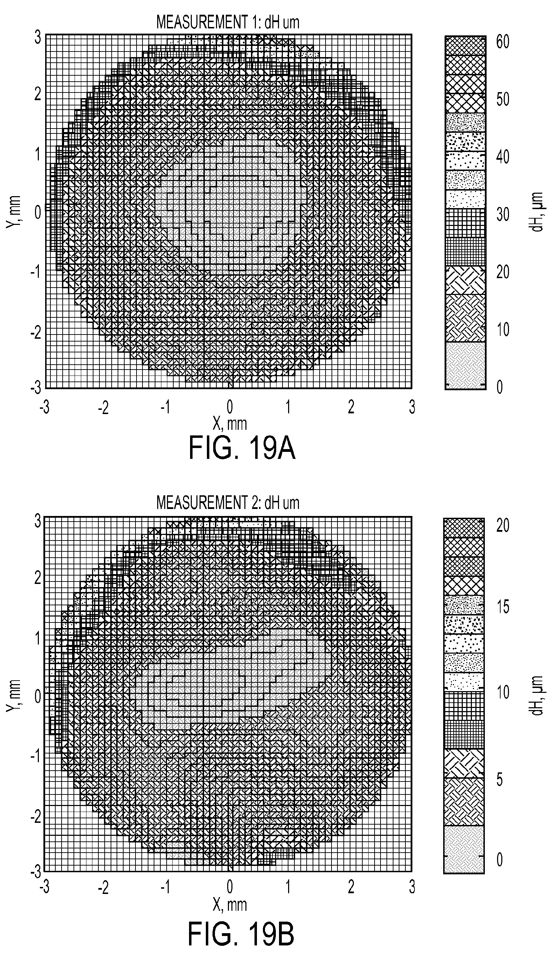

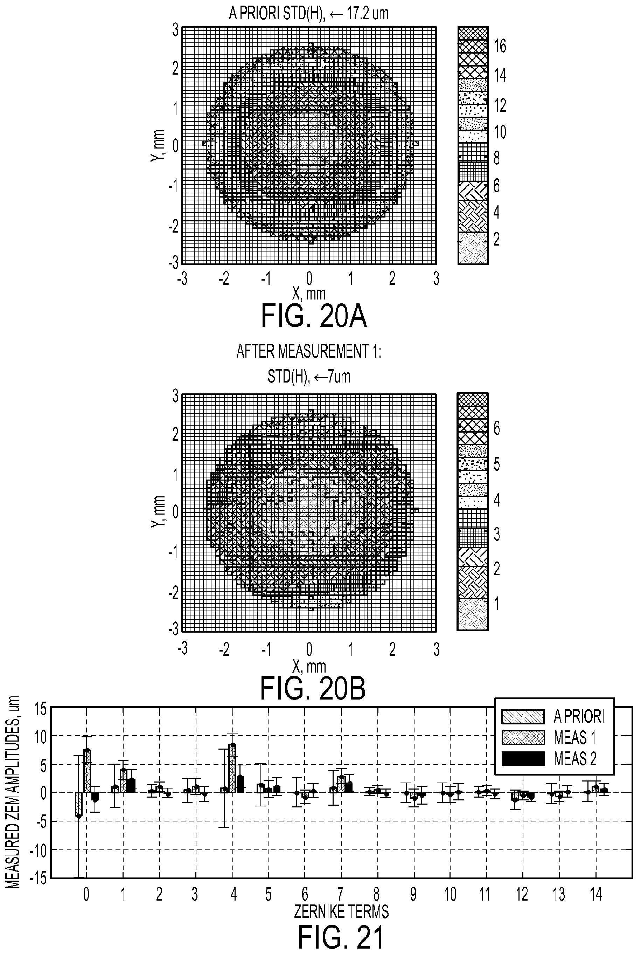

Estimated Zernike amplitudes allow reconstruction of the corneal elevation field within the circular area used for Zernike decomposition. FIGS. 19A and 19B are graphs illustrating deviations of the corneal elevation measurements shown in FIGS. 18A and 18B from an average corneal shape, respectively. The estimated covariance matrix allows for an estimate of the uncertainty of corneal elevations and Zernike amplitudes. FIG. 20A is a graph illustrating corneal elevation uncertainty for an *a priori* measured corneal shape. FIG. 20B is a graph illustrating corneal elevation uncertainty for a post-measurement corneal shape. FIG. 21 is a chart illustrating *a priori* and measured Zernike amplitudes.

Applying the Kalman-Bucy technique, two estimates of measured values weighted by respective variances are combined. As a result, larger random deviations from the prior estimate make relatively smaller contribution to the measurement result. The measurement results are thus more robust and statistically optimal. This method extracts maximum available information from all of the measurements combined as well as from *a priori* knowledge of the measured field.

Data assimilation, based on the Kalman-Bucy algorithm, has the following advantages compared to a simple averaging of measured data: statistically optimal combination of multiple measurements; assimilation of a priori information; protection from measurement outliers; and an objective estimate of measurement accuracy.

## Statistics of human corneal topography

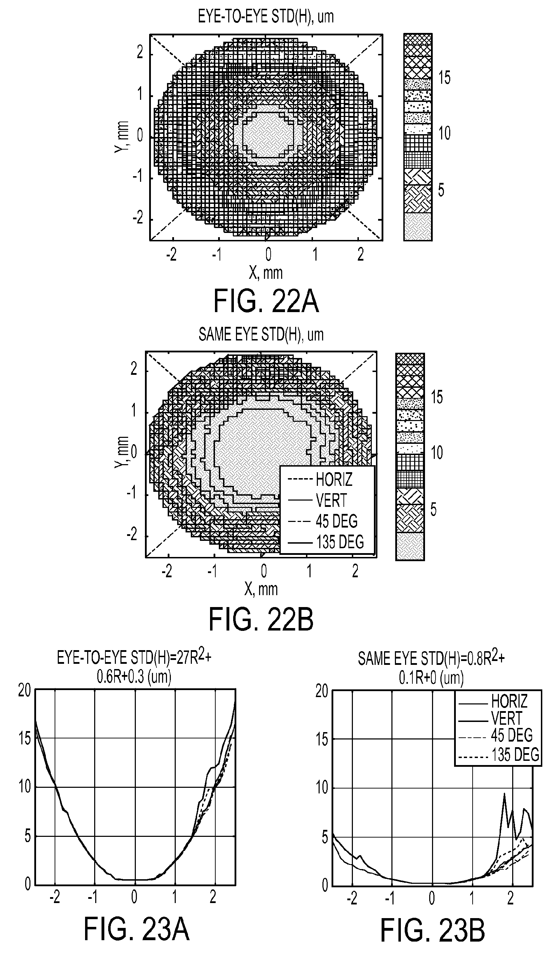

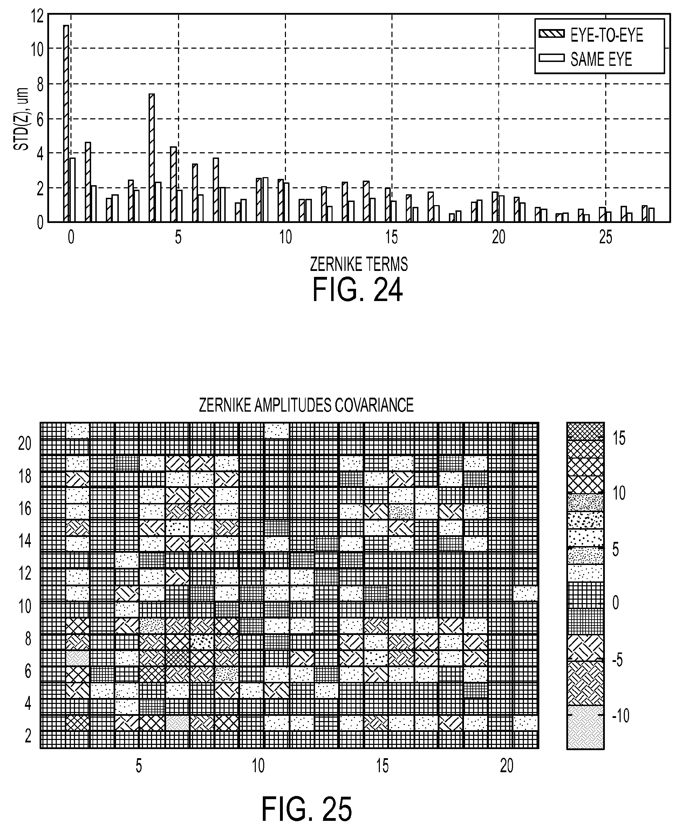

Corneal topography data from a population of pre-operational eyes, some of which had multiple measurements, can be used to determine statistics for two types of random fluctuations of measurement data: variations of multiple measurements for the same; and eye-to-eye differences (variations of measurement averages between different eyes). FIG. 22A is graph illustrating standard deviation of a corneal elevation field for different eyes based on variations of measurement averages between the different eyes. FIG. 22B is a graph illustrating standard deviation of a corneal elevation field for multiple measurements of the same eye. FIG. 23A is a graph illustrating radial dependence for the standard deviation of corneal elevation shown in FIG. 22A. FIG. 23B is a graph illustrating radial dependence for the standard deviation of corneal elevation shown in FIG. 22B. FIG. 24 is a chart illustrating standard deviation of Zernike amplitudes for the corneal elevation fields shown in FIGS. 22A and 22B. FIG. 25 is a graph illustrating covariance of Zernike amplitudes for corneal elevation.

## Kalman-Bucy Technique for Corneal Topography Data Processing

In one embodiment, a method of sequential assimilation of corneal topography measurements combines each measurement of a corneal elevation field with results of previous topography measurements (e.g., archived data) for the same eye and includes a *priori* statistical information of corneal shape. For each corneal topography studied, the Kalman-Bucy technique yielded an estimate of Zernike amplitudes and a corresponding covariance matrix. These estimates resulted in a reconstructed corneal elevation map without gaps within a circular area used for Zernike decomposition, as well as a map of measurement uncertainty.

The sequential estimation method is designed to combine a *priori* statistical information with one or more measurements of a system, described by a $M \times 1$ parameter vector, *A.* We assume that the system is governed by a linear system

$$h = \hat{G}A + \widetilde{h} \, , \qquad\qquad\qquad (B1)$$

where *h* is the D x 1 vector of observations, G is the coefficients matrix of a linear observation model, and $\widetilde{h}$ is the vector of measurement errors.

For corneal topography measurements the corneal elevation shall be the vector of observations, *h*, and Zernike decomposition of elevation field is used as a linear model:

$$h(x,y) = \sum_{i=0}^{N} A_i \cdot Z_i(x,y) \, , \qquad\qquad (B2)$$

where $Z_i(x, y)$ are Zernike polynomials and $A_i$ are the amplitudes of Zernike decomposition.

Thus if the elevation field is defined on a set of points, $(x_k, y_k)$, the model will be defined by the $M \times M$ matrix,

$$G_{i,k} = Z_i(x_k, y_k) . \qquad (B3)$$

The Kalman-Bucy algorithm, applied to corneal topography measurements, assimilates each measurement by combining the measured data (cornea elevations field) with the prior estimate of mean vector, $A_i^{(j)}$, and covariance matrix for Zernike amplitudes, $C_{ik}^{(j)} \equiv \left\langle A_i^{(j)} \cdot A_k^{(j)} \right\rangle$, as defined by the following formulas:

$$A_i^{(j+1)} = A_i^{(j)} + \hat{K}^{(j)} \cdot \left[ h_k^{(j)} - G_{i,k} \cdot A_i^{(j)} \right] \qquad (B4)$$

$$\hat{C}^{(j+1)} = \left\{ \hat{I} - \hat{K}^{(j)} \cdot \hat{G}^{(j)} \right\} \cdot \hat{C}^{(j)} , \qquad (B5)$$

where $I$ is the identity matrix having dimensions $M \times M$,

$$\hat{K}^{(j)} = \hat{C}^{(j)} \cdot G^T \cdot \hat{F}^{(j)} \qquad (B6)$$

is the Kalman-Bucy gain, and

$$\hat{F}^{(j)} = \left\{ \hat{G} \cdot \hat{C}^{(j)} \cdot \hat{G}^T + \hat{N} \right\}^{-1} \qquad (B7)$$

is a D x D matrix. The $D \times D$ matrix $\hat{N}$ is the covariance matrix of measurement errors.

Formulas (B4-B7) provide a statistical optimal estimation of the parameter vector, $A_i^{(j+1)}$, and its covariance matrix, $\hat{C}^{(j+1)}$, after the $j$-th measurement is assimilated together with prior $j$-1 measurements.

This method gives us an iterative algorithm of sequential assimilation of corneal topography measurements. The first step in the iterative sequence starts with an a *priori* estimate of mean parameter vector (Zernike amplitudes of corneal elevation) and the corresponding covariance matrix. These a *priori* values for corneal topography, as well as the covariance matrix N of the elevations measurement errors, can be derived from statistics of general population, as previously mentioned.

Once we have the covariance matrix for Zernike amplitudes, we can calculate variance of the corneal elevation field as follows:

$$Var(h(x,y)) = \left\langle \left( \sum_i A_i \cdot Z_i(x,y) \right) \cdot \left( \sum_k A_k \cdot Z_k(x,y) \right) \right\rangle$$
$$= \sum_{i,k} C_{i,k} \cdot Z_i(x,y) \cdot Z_k(x,y) \qquad (B8)$$

**Example 1**

With many topography fields measured for normal human eyes we can decompose each elevation field into Zernike series and evaluate the mean and variance of each amplitude. This will be the a priori information to input into the Kalman-Bucy filter together with the measurement data. The Kalman-Bucy filter will be applied according to the following formulas:

$$A_k^+ = A_k^- + \hat{K} \cdot \left\{ \bar{H} - \hat{G} \cdot A_k^- \right\}, \ M_k^+ = \hat{K} \cdot \left\{ \hat{I} - \hat{K}\hat{G} \right\} \cdot M_k^-$$

Here $A_k^-, M_k^-$ and $A_k^+, M_k^+$ are the Zernike amplitudes and their covariance matrices prior and after assimilation of measurement data, respectively, $\vec{H}$ is the vector of measurement data (topography elevations), $\hat{G}$ is the operator of surface reconstruction from Zernike amplitudes (a surface defined on $x_i$, $y_i$ points can be computed from Zernike amplitudes and Zernike polynomials $\Phi_k(x_i, y_i)$ at these points as $S_i = \sum A_k \Phi_k(x_i, y_i)$)). $\hat{K} = \hat{M}^{-1}\hat{G}^T\hat{F}$ is the Kalman-Bucy gain, $\hat{F} = \{\hat{G}M^-\hat{G}^T + \hat{N}\}^{-1}$, $\hat{N}$ is the data noise covariance matrix, and $\hat{I}$ is a unitary matrix.

Sequential measurements shall apply the same procedure to the prior measurement outcome, $A_k^-, M_k^-$. As a result, with each measurement assimilated the measured amplitudes approach closer to the true value and the measurement uncertainty decreases. Diagonal elements of the covariance matrix, $\hat{M}$, give us the estimate of uncertainty in the measured amplitude.

In some embodiments, the same method of sequential estimation can be applied to wavefront measurements, performed with an aberrometer, where a lenslet images of distorted spots yield noisy and incomplete data. Multiple measurements and Fourier decomposition are typically used there to re-construct the wavefront surface. Sequential estimation can provide a statistically optimal combination of sequential measurements and include a *priori* information. In some embodiments, the same method of sequential estimation can be applied to combinations of different measurements, such as wavefront and topography measurements.

While the above provides a complete and accurate description of specific embodiments of the invention, several changes and adaptations of the present invention may be readily made. While specific reference has been made to correcting optical aberrations made with refractive, wavefront and topography measurements, methods and systems of the present invention can be used to ablate any desired shape in tissue based on any measurement. While specific reference has been made to using Zernike polynomials with Kalman-Bucy technique, a different set of orthogonal polynomials of Fourier decomposition may be used in place of Zernike polynomials.

**Claims**

1. A method of measuring a topography of an optical tissue surface of an eye, the method comprising:

   combining measured elevations of the surface with a *priori* information of the surface to provide an estimate of mean and covariance of post-measurement orthogonal polynomial sequence amplitudes associated with the surface, the a *priori* information comprising an estimate of mean and covariance of pre-measurement orthogonal polynomial sequence amplitudes associated with the surface;
   determining a variance of elevation of the surface from the estimate of mean and covariance of post-measurement amplitudes associated with the surface, the variance representing a measure of measurement quality; and
   constructing the topography from the estimate of mean and covariance of post- measurement amplitudes based on a comparison of the variance of elevation of the surface with a pre-determined threshold.

2. The method according to claim 1, wherein the optical tissue surface is selected from a group consisting of a corneal surface of the eye and a wavefront of the eye.

3. The method according to claim 1, wherein the optical tissue surface is a corneal surface of the eye, the method further comprising:

   measuring a plurality of elevations for a corneal surface;
   wherein the post-measurement orthogonal polynomial sequence amplitudes are post-measurement Zernike amplitudes, wherein the estimate of mean and covariance of pre-measurement orthogonal polynomial sequence amplitudes is a plurality of mean and covariance of pre-measurement Zernike amplitudes associated with the corneal surface known prior to the measuring step.

4. The method according to claim 3, further comprising estimating the plurality of mean and covariance of Zernike amplitudes associated with the corneal surface prior to the measuring step.

5. The method of claim 3 or 4, further comprising decomposing each of the measured elevations into a Zernike series representation.

6. The method of any of claims 3 to 5, further comprising:

acquiring topography elevation fields measured of human eyes;
decomposing each of the topography elevation fields into a Zernike series; evaluating a mean and a variance of each amplitude of the Zernike series; and preparing the a priori information from the mean and the variance of each amplitude of the Zernike series.

7. The method of any of claims 3 to 6, wherein the combining step comprises inputting the a priori information into a Kalman-Bucy filter together with the measured elevations.

8. The method of claim 7, wherein the inputting step comprises applying the Kalman -Bucy filter according to

$$A_k^+ = A_k^- + \hat{K} \cdot \left\{ \vec{H} - \hat{G} \cdot A_k^- \right\}, \quad M_k^+ = \hat{K} \cdot \left\{ \hat{I} - \hat{K}\hat{G} \right\} \cdot M_k^-$$

$$\hat{K} = \hat{M}^{-1} \hat{G}^T \hat{F}$$

$$\hat{F} = \left\{ \hat{G} M^- \hat{G}^T + \hat{N} \right\}^{-1}$$

where $A_k^-$ represents Zernike amplitudes prior to assimilation of measurement data, $M_k^-$ is a covariance matrix of $A_k^-$, $A_k^+$ represents Zernike amplitudes after assimilation of measurement data, $M_k^+$ is a covariance matrix of $A_k^+$, $\vec{H}$ is a vector of the measured elevations, $\hat{G}$ is an operator of surface reconstruction from the Zernike amplitudes, $\hat{K}$ is a Kalman-Bucy gain, $\hat{F}$ is a $D \times D$ matrix, $\hat{N}$ is a data noise covariance matrix, $\hat{M}$ is covariance matrix of the measured amplitudes, and $\hat{I}$ is a unitary matrix.

9. A method of planning a refractive correction treatment for an eye, the method comprising:

measuring a plurality of elevations for a corneal surface of the eye;
combining the measured elevations with a priori information of the corneal surface to provide an estimate of mean and covariance of post-measurement Zernike amplitudes associated with the corneal surface, the a priori information comprising a plurality of mean and covariance of pre-measurement Zernike amplitudes associated with the corneal surface known prior to the measuring step;
determining a variance of elevation of the corneal surface from the estimate;
constructing the topography of the corneal surface from the estimate based on a comparison of the variance with a pre-determined threshold;
determining ablation properties locally across the corneal surface based on the topography; and
formulating a treatment plan using the ablation properties by adjusting a first virtual ablation shape to form a second virtual ablation shape, the first virtual shape representing a depth of material to be removed from the treatment area to form a desired shape, the second virtual shape being formed from the first virtual shape in response to the topography.

10. A system for treating a corneal surface of a patient's eye with a laser beam, the eye having a refractive defect, wherein a desired refractive correcting shape mitigates the refractive defect, the system comprising:

a laser emitting a beam of an ablative light energy; and at least one processor coupled to the laser beam and having a computer program, the computer program embodying instructions for:

combining measured elevations of the corneal surface with a priori information of the corneal surface to provide an estimate of mean and covariance of post-measurement Zernike amplitudes associated with the corneal surface, the a priori information comprising a plurality of mean and covariance of pre- measurement Zernike amplitudes associated with the corneal surface known prior to the measuring step;
determining a variance of elevation of the corneal surface from the estimate;
constructing the topography of the corneal surface from the estimate based on a comparison of the variance with a pre-determined threshold;

determining ablation properties locally across the corneal surface based on the topography;
formulating a treatment plan using the ablation properties by adjusting a first virtual ablation shape to form a second virtual ablation shape, the first virtual shape representing a depth of material to be removed from the treatment area to form a desired shape, the second virtual shape being formed from the first virtual shape in response to the topography; and
controlling an ablative treatment using the treatment plan from the second virtual shape so that the treatment forms the desired refractive correcting shape in the surface.

## Patentansprüche

1. Verfahren zum Messen einer Topographie einer optischen Gewebeoberfläche eines Auges, wobei das Verfahren umfasst:

   Kombinieren gemessener Höhenwerte der Oberfläche mit *a-priori*-Informationen über die Oberfläche, um eine Abschätzung von Mittelwert und Kovarianz von mit der Oberfläche verbundenen orthogonalen Polynomsequenzamplituden von nach der Messung zu erhalten, wobei die *a-priori*-Informationen eine Abschätzung von Mittelwert und Kovarianz von mit der Oberfläche verbundenen orthogonalen Polynomsequenzamplituden von vor der Messung umfassen;
   Bestimmen einer Varianz von Höhenwerten der Oberfläche aus der Abschätzung von Mittelwert und Kovarianz von mit der Oberfläche verbundenen Amplituden von nach der Messung, wobei die Varianz ein Maß der Messqualität darstellt; und
   Erstellen der Topographie aus der Abschätzung von Mittelwert und Kovarianz von Amplituden von nach der Messung auf der Grundlage eines Vergleichs der Varianz von Höhenwerten der Oberfläche mit einem vorbestimmten Schwellenwert.

2. Verfahren gemäß Anspruch 1, wobei die optische Gewebeoberfläche ausgewählt ist aus einer Gruppe bestehend aus einer Hornhautoberfläche des Auges und einer Wellenfront des Auges.

3. Verfahren gemäß Anspruch 1, wobei die optische Gewebeoberfläche eine Hornhautoberfläche des Auges ist, wobei das Verfahren ferner umfasst:

   Messen einer Vielzahl von Höhenwerten für eine Hornhautoberfläche;
   wobei die orthogonalen Polynomsequenzamplituden von nach der Messung Zernike-Amplituden von nach der Messung sind, wobei die Abschätzung von Mittelwert und Kovarianz von orthogonalen Polynomsequenzamplituden von vor der Messung eine Vielzahl von Mittelwerten und Kovarianzen von mit der Hornhautoberfläche verbundenen Zernike-Amplituden von vor der Messung sind, die vor dem Messschritt bekannt sind.

4. Verfahren gemäß Anspruch 3, ferner umfassend Abschätzen der Vielzahl von Mittelwerten und Kovarianzen von mit der Hornhautoberfläche verbundenen Zernike-Amplituden vor dem Messschritt.

5. Verfahren gemäß Anspruch 3 oder 4, ferner umfassend Zerlegen jeder der gemessenen Höhenwerte in eine Zernike-Polynomdarstellung.

6. Verfahren gemäß einem der Ansprüche 3 bis 5, ferner umfassend:

   Aufnehmen von Topographie-Höhenwertfeldern, die an menschlichen Augen gemessen sind;
   Zerlegen jedes der Topographie-Höhenwertfelder in ein Zernike-Polynom; Auswerten von Mittelwert und Varianz jeder Amplitude des Zernike-Polynoms; und Erstellen der *a-priori*-Informationen aus dem Mittelwert und der Varianz jeder Amplitude des Zernike-Polynoms.

7. Verfahren gemäß einem der Ansprüche 3 bis 6, wobei der Schritt des Kombinierens Eingeben der *a-priori*-Informationen in ein Kalman-Bucy-Filter zusammen mit den gemessenen Höhenwerten umfasst.

8. Verfahren gemäß Anspruch 7, wobei der Schritt des Eingebens Anwenden des Kalman-Bucy-Filters gemäß

$$A_k^+ = A_k^- + \hat{K} \cdot \{\vec{H} - \hat{G} \cdot A_k^-\}, \quad M_k^+ = \hat{K} \cdot \{\hat{I} - \hat{K}\hat{G}\} \cdot M_k^-$$

$$\hat{K} = \hat{M}^{-1}\hat{G}^T\hat{F}$$

$$\hat{F} = \{\hat{G}M^-\hat{G}^T + \hat{N}\}^{-1}$$

umfasst, wobei $A_k^-$ Zernike-Amplituden vor der Angleichung von Messdaten darstellt, $M_k^-$ eine Kovarianzmatrix von $A_k^-$ ist, $A_k^+$ Zernike-Amplituden nach der Angleichung von Messdaten darstellt, $M_k^+$ eine Kovarianzmatrix von $A_k^+$ ist, $\vec{H}$ ein Vektor der gemessenen Höhenwerte ist, $\hat{G}$ ein Operator der Oberflächenrekonstruktion aus den Zernike-Amplituden ist, $\hat{K}$ eine Kalman-Bucy-Verstärkung ist, $\hat{F}$ eine D x D-Matrix ist, $\hat{N}$ eine Daten-Rausch-Kovarianzmatrix ist, $\hat{M}$ die Kovarianzmatrix der gemessenen Amplituden ist und $\hat{I}$ eine unitäre Matrix ist.

9. Verfahren zum Planen einer Brechungskorrekturbehandlung für ein Auge, wobei das Verfahren umfasst:

Messen einer Vielzahl von Höhenwerten für eine Hornhautoberfläche des Auges;
Kombinieren der gemessenen Höhenwerte mit *a-priori*-Informationen über die Hornhautoberfläche, um eine Abschätzung von Mittelwert und Kovarianz von mit der Hornhautoberfläche verbundenen Zernike-Amplituden von nach der Messung zu erhalten, wobei die *a-priori*-Informationen eine Vielzahl von Mittelwerten und Kovarianzen von mit der Hornhautoberfläche verbundenen Zernike-Amplituden von vor der Messung umfassen, die vor dem Messschritt bekannt sind;
Bestimmen einer Varianz von Höhenwerten der Hornhautoberfläche aus der Abschätzung;
Erstellen der Topographie der Hornhautoberfläche aus der Abschätzung auf der Grundlage eines Vergleichs der Varianz mit einem vorbestimmten Schwellenwert;
Lokales Bestimmen von Ablationseigenschaften über die Hornhautoberfläche auf der Grundlage der Topographie; und
Formulieren eines Behandlungsplans unter Verwendung der Ablationseigenschaften durch Einstellen einer ersten virtuellen Ablationsform, um eine zweite virtuelle Ablationsform zu bilden, wobei die erste virtuelle Form eine Tiefe von Material darstellt, das von dem Behandlungsbereich entfernt werden soll, um eine gewünschte Form zu bilden, und die zweite virtuelle Form aus der ersten virtuellen Form in Antwort auf die Topographie gebildet wird.

10. System zum Behandeln einer Hornhautoberfläche eines Auges eines Patienten mit einem Laserstrahl, wobei das Auge einen Brechungsdefekt aufweist, wobei eine gewünschte brechungskorrigierende Form den Brechungsdefekt mildert, wobei das System umfasst:

einen Laser, der einen Strahl mit einer ablativen Lichtenergie emittiert; und
wenigstens einen Prozessor, der an den Laserstrahl gekoppelt ist und ein Computerprogramm aufweist, wobei das Computerprogramm Anweisungen verkörpert zum:

Kombinieren gemessener Höhenwerte der Hornhautoberfläche mit *a-priori*-Informationen über die Hornhautoberfläche, um eine Abschätzung von Mittelwert und Kovarianz von mit der Hornhautoberfläche verbundenen Zernike-Amplituden von nach der Messung zu erhalten, wobei die *a-priori*-Informationen eine Vielzahl von Mittelwerten und Kovarianzen von mit der Hornhautoberfläche verbundenen Zernike-Amplituden von vor der Messung umfassen, die vor dem Messschritt bekannt sind;
Bestimmen einer Varianz von Höhenwerten der Hornhautoberfläche aus der Abschätzung;
Erstellen der Topographie der Hornhautoberfläche aus der Abschätzung auf der Grundlage eines Vergleichs der Varianz mit einem vorbestimmten Schwellenwert;
Lokales Bestimmen von Ablationseigenschaften über die Hornhautoberfläche auf der Grundlage der Topographie;
Formulieren eines Behandlungsplans unter Verwendung der Ablationseigenschaften durch Einstellen einer ersten virtuellen Ablationsform, um eine zweite virtuelle Ablationsform zu bilden, wobei die erste virtuelle Form eine Tiefe von Material darstellt, das von dem Behandlungsbereich entfernt werden soll, um eine gewünschte Form zu bilden, und die zweite virtuelle Form aus der ersten virtuellen Form in Antwort auf die Topographie gebildet wird; und

Steuern einer Ablationsbehandlung unter Verwendung des Behandlungsplans aus der zweiten virtuellen Form, so dass die Behandlung die gewünschte brechungskorrigierende Form in der Oberfläche bildet.

**Revendications**

1. Procédé de mesure d'une topographie d'une surface de tissu optique d'un oeil, le procédé comprenant les étapes suivantes :

   combiner des élévations mesurées de la surface avec des informations a priori de la surface pour fournir une estimation de la moyenne et de la covariance d'amplitudes de séquence polynomiale orthogonale après mesure associées à la surface, les informations a priori comprenant une estimation de la moyenne et de la covariance d'amplitudes de séquence polynomiale orthogonale avant mesure associées à la surface ;
   déterminer une variance de l'élévation de la surface à partir de l'estimation de la moyenne et de la covariance d'amplitudes après mesure associées à la surface, la variance représentant une mesure de la qualité de mesure ; et
   construire la topographie à partir de l'estimation de la moyenne et de la covariance d'amplitudes après mesure sur la base d'une comparaison de la variance de l'élévation de la surface avec un seuil prédéterminé.

2. Procédé selon la revendication 1, dans lequel la surface de tissu optique est sélectionnée dans un groupe consistant en une surface de cornée de l'oeil et un front d'onde de l'oeil.

3. Procédé selon la revendication 1, dans lequel la surface de tissu optique est une surface de cornée de l'oeil, le procédé comprenant en outre les étapes suivantes :

   mesurer une pluralité d'élévations pour une surface de cornée ;
   où les amplitudes de séquence polynomiale orthogonale après mesure sont des amplitudes de Zernike après mesure, où l'estimation de la moyenne et de la covariance d'amplitudes de séquence polynomiale orthogonale avant mesure est une pluralité de moyennes et de covariances d'amplitudes de Zernike avant mesure associées à la surface de cornée connue avant l'étape de mesure.

4. Procédé selon la revendication 3, comprenant en outre une estimation de la pluralité de moyennes et de covariances d'amplitudes de Zernike associées à la surface de cornée avant l'étape de mesure.

5. Procédé selon la revendication 3 ou la revendication 4, comprenant en outre de décomposer chacune des élévations mesurées en une représentation en série de Zernike.

6. Procédé selon l'une quelconque des revendications 3 à 5, comprenant en outre les étapes suivantes :

   acquérir des champs d'élévation de topographie mesurés de yeux humains ; décomposer chacun des champs d'élévation de topographie en une série de Zernike ;
   évaluer une moyenne et une variance de chaque amplitude de la série de Zernike ; et préparer les informations a priori à partir de la moyenne et de la variance de chaque amplitude de la série de Zernike.

7. Procédé selon l'une quelconque des revendications 3 à 6, dans lequel l'étape de combinaison comprend d'entrer les informations a priori dans un filtre de Kalman-Bucy avec les élévations mesurées.

8. Procédé selon la revendication 7, dans lequel l'étape d'entrer comprend d'appliquer le filtre de Kalman-Bucy conformément aux équations suivantes :

$$A_k^+ = A_k^- + \hat{K}.\{\vec{H} - \hat{G}.A_k^-\}, \quad M_k^+ = \hat{K}.\{\hat{I} - \hat{K}\hat{G}\}.M_k^-$$

$$\hat{K} = \hat{M}^{-1}\hat{G}^T\hat{F}$$

$$\hat{F} = \left\{ \hat{G} M^- \hat{G}^T + \hat{N} \right\}^{-1}$$

où $A_k^-$ représente des amplitudes de Zernike avant assimilation des données de mesure, $M_k^-$ est une matrice de covariance de $A_k^-$, $A_k^+$ représente des amplitudes de Zernike après assimilation des données de mesure, $M_k^+$ est une matrice de covariance de $A_k^+$, $\vec{H}$ est un vecteur des élévations mesurées, $\hat{G}$ est un opérateur de reconstruction de surface à partir des amplitudes de Zernike, $\hat{K}$ est un gain de Kalman-Bucy, $\hat{F}$ est une matrice DxD, $\hat{N}$ est une matrice de covariance de bruit de données, $\hat{M}$ est une matrice de covariance des amplitudes mesurées, et $\hat{I}$ est une matrice unité.

9. Procédé de planification d'un traitement de correction de réfraction pour un oeil, le procédé comprenant les étapes suivantes :

mesurer une pluralité d'élévations pour une surface de cornée de l'oeil ;
combiner les élévations mesurées avec des informations a priori de la surface de la cornée pour fournir une estimation de la moyenne et de la covariance d'amplitudes de Zernike après mesure associées à la surface de la cornée, les informations a priori comprenant une pluralité de moyennes et de covariances d'amplitudes de Zernike avant mesure associées à la surface de la cornée connue avant l'étape de mesure ;
déterminer une variance de l'élévation de la surface de la cornée à partir de l'estimation ;
construire la topographie de la surface de la cornée à partir de l'estimation sur la base d'une comparaison de la variance avec un seuil prédéterminé ;
déterminer des propriétés d'ablation localement à travers la surface de la cornée sur la base de la topographie ; et
formuler un programme de traitement au moyen des propriétés d'ablation en ajustant une première forme d'ablation virtuelle pour former une seconde forme d'ablation virtuelle, la première forme d'ablation virtuelle représentant une profondeur de matériau à retirer de la zone de traitement pour former une forme souhaitée, la seconde forme virtuelle étant formée à partir de la première forme virtuelle en réponse à la topographie.

10. Système de traitement d'une surface de cornée de l'oeil d'un patient avec un faisceau laser, l'oeil présentant un défaut de réfraction, où une forme de correction de la réfraction souhaitée atténue le défaut de réfraction, le système comprenant :

un laser émettant un faisceau d'énergie lumineuse ablative ; et au moins un processeur couplé au faisceau laser et ayant un programme informatique, le programme informatique intégrant des instructions pour :

combiner des élévations mesurées de la surface de la cornée avec des informations a priori de la surface de la cornée pour fournir une estimation de la moyenne et de la covariance d'amplitudes de Zernike associées à la surface de la cornée, les informations a priori comprenant une pluralité de moyennes et de covariances d'amplitudes de Zernike avant mesure associées à la surface de la cornée avant l'étape de mesure ;
déterminer une variance de l'élévation de la surface de la cornée à partir de l'estimation ;
construire la topographie de la surface de la cornée à partir de l'estimation sur la base d'une comparaison de la variance avec un seuil prédéterminé ;
déterminer des propriétés d'ablation localement à travers la surface de la cornée sur la base de la topographie ;
formuler un programme de traitement au moyen des propriétés d'ablation en ajustant une première forme d'ablation virtuelle pour former une seconde forme d'ablation virtuelle, la première forme virtuelle représentant une profondeur de matériau à retirer de la zone de traitement pour former une forme souhaitée, la seconde forme virtuelle étant formée à partir de la première forme virtuelle en réponse à la topographie ; et
contrôler un traitement d'ablation au moyen du programme de traitement à partir de la seconde forme virtuelle de manière à ce que le traitement forme la forme de correction de réfraction souhaitée dans la surface.

FIG. 1

FIG. 1A

FIG. 2

FIG. 3

FIG. 4

139 — LASER BEAM ANGLES

OPTICAL SURFACES — 134

CORNEAL TOPOGRAPHY

137

ABLATIVE PULSE CHARACTERISTICS

138

Tx PROGRAM — 136

TREATMENT TABLE

52

LASER ABLATION

142

FIG. 5

140

PATIENT NAME ⌐150
REPRATE ⌐152 ⌐154
PATIENT ID ⌐156
EYE ⌐158    OD
REFRACTION    1079    -3    ⌐160 -2.25    60    0
PULSE COUNT
PULSE NO.

| PULSE NO. | IRIS DIAM | SLIT WIDTH | SLIT AXIS | X (mm) | Y (mm) |
|---|---|---|---|---|---|
| 1 | 1.5 | 6.5 | 0 | -0.4 | 0.2 |
| 2 | 1.5 | 6.5 | 0 | 0.3 | -0.6 |
| 3 | 1.5 | 6.5 | 0 | -0.1 | 0 |
| 4 | 1.5 | 6.5 | 0 | 0.2 | 1 |
| 5 | 1.5 | 6.5 | 0 | -0.3 | -0.8 |
| 6 | 1.5 | 6.5 | 0 | 1.7 | 0.1 |
| 7 | 1.5 | 6.5 | 0 | 0.6 | -1.5 |
| 170 | 172 | 174 | 176 | 178 | 180 |

FIG. 6

FIG. 7

FIG. 8

FIG. 8A

FIG. 8B

FIG. 9

FIG. 9A

FIG. 10

FIG. 10A

FIG. 11

FIG. 12

FIG. 13

FIG. 14

FIG. 15

FIG. 16

FIG. 17

MEASUREMENT 1: H, um

FIG. 18A

MEASUREMENT 2: H, um

FIG. 18B

FIG. 19A

FIG. 19B

A PRIORI STD(H), ← 17.2 um

FIG. 20A

AFTER MEASUREMENT 1:

STD(H), ←7um

FIG. 20B

FIG. 21

EYE-TO-EYE STD(H), um

## FIG. 22A

SAME EYE STD(H), um

- - - - HORIZ
——— VERT
— - — 45 DEG
——— 135 DEG

## FIG. 22B

EYE-TO-EYE STD(H)=27R$^2$+
0.6R+0.3 (um)

## FIG. 23A

SAME EYE STD(H)=0.8R$^2$+
0.1R+0 (um)

——— HORIZ
——— VERT
— - — 45 DEG
- - - - 135 DEG

## FIG. 23B

FIG. 24

ZERNIKE AMPLITUDES COVARIANCE

FIG. 25

## REFERENCES CITED IN THE DESCRIPTION

**Patent documents cited in the description**

- US 5163934 A
- US 4761071 A
- US 4995716 A
- US 5406342 A
- US 6396069 B
- US 6116738 A
- US 4540254 A
- US 5491524 A
- US 6245059 B
- US 6129722 A
- US 5843070 A
- US 5777719 A
- US 6042012 A
- US 6095651 A
- US 6199986 B
- US 6271914 B
- US 6217915 B
- US 5144630 A

- US 5742626 A
- US 5713892 A
- US 5683379 A
- US 96838097 A
- US 09274999 B
- US 4665913 A
- US 46889895 A
- US 6319247 B
- US 6280435 B
- US 6203539 B
- US 5646791 A
- US 5912779 A
- US 6331177 B
- US 5912775 A
- US 6673062 B
- US 7008415 B
- US 6315413 B
- US 6302876 B

**Non-patent literature cited in the description**

- **WEAAM F. M. ALKHALDI.** *Statistical Signal and Image Processing Techniques in Corneal Modeling,* 13 July 2010, http://tuprints.ulb.tu-darmstadt.de/2232/2/WeaamAlkhaldi_PhD_Thesis.pdf

- **BORSUZTKY et al.** Tunable UV Radiation at Short Wavelengths (188-240 nm) Generated by Sum Frequency Mixing in Lithium Borate. *Appl. Phys.,* 1995, vol. 61, 529-532